# EUROPEAN PATENT APPLICATION

(11) **EP 4 563 572 A1**
(43) Date of publication of application: **04.06.2025**
(21) Application number: 23846490.3
(22) Date of filing: 25.07.2023
(51) Int. Cl.: C07D 231/20, C07D 231/22, C07D 401/04, C07D 403/04, C07H 1/00, C07H 7/06

(54) **PYRAZOLONE COMPOUND AND PYRAZOLONE AGENT**

(30) Priority: 25.07.2022 JP 2022118259
(71) Applicant: The Noguchi Institute, Tokyo 1730003 (JP)
(72) Inventor: OSUMI Kenji, Tokyo 173-0003 (JP); GOTO Kohtaro, Tokyo 173-0003 (JP); TSUTSUI Masato, Tokyo 173-0003 (JP); MIZUNO Mamoru, Tokyo 173-0003 (JP); SUGI Tomokazu, Tokyo 173-0003 (JP)
(74) Representative: Gille Hrabal Partnerschaftsgesellschaft mbB Patentanwälte
(86) International application number: PCT/JP2023/027102
(87) International publication number: WO 2024/024760

(57) **Abstract**

The object of present invention is to provide pyrazolonated compounds with high stability.

The object can be solved by a pyrazolone compound represented by the following formula (1): 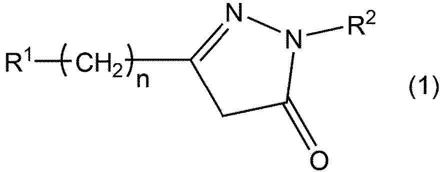 wherein R¹ is an electron withdrawing group selected from the group consisting of one or more selected from the group consisting of a halogen atom, a nitro group, a cyano group, a haloalkyl group having 1 to 10 carbon atoms, a perfluoropolyether group having 2 to 10 carbon atoms, and the following formulas (2) to (7): 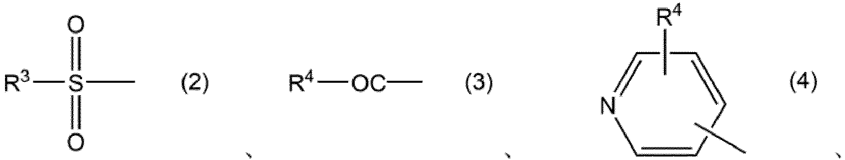 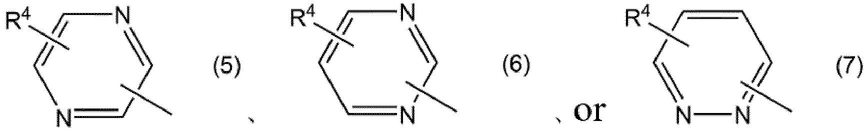 R² is a hydrocarbon group having 1 to 1000 carbon atoms, which may contain a heteroatom, and n is an integer of 0 to 2.

## Description

### TECHNICAL FIELD

The present invention relates to the pyrazolone compound and the pyrazolonating agent.

### BACKGROUND ART

It is known that the formyl groups of proteins such as antibodies can be modified with pyrazolone compounds to introduce fluorescent molecules (Patent literature 1 and Non-patent literatures 1 to 3).

### CITATION LIST

### PATENT LITERATURE

[PATENT LITERATURE 1] WO 2014/074218

### NON-PATENT LITERATURE

[NON-PATENT LITERATURE 1] ACS Medicinal Chemistry Letters (U.S.A.) 2016, Vol. 7, p. 994-998
[NON-PATENT LITERATURE 2] ChemBioChem (Germany) 2020, Vol. 21, p. 3580-3593
[NON-PATENT LITERATURE 3] ChemSusChem (Germany) 2022, e202102592

### SUMMARY OF INVENTION

### TECHNICAL PROBLEM

The present inventors have prepared pyrazolonated compounds by modifying the formyl group of sugar using 3-methyl-1-phenyl-5-pyrazolone as a pyrazolone compound. However, the obtained pyrazolonated compounds were found to be easily decomposed and have low stability.

Therefore, the object of present invention is to provide pyrazolonated compounds with high stability.

### SOLUTION TO PROBLEM

In an intensive studies for a pyrazolonated compound with high stability, the present inventors found surprising results that a pyrazolonated compound with high stability can be obtained by modify a formyl group using pyrazolone in which an electron withdrawing group is introduced in specific positions.

The present invention is based on the above findings.

Therefore, the present invention relates to
[1] a pyrazolone compound represented by the following formula (1): 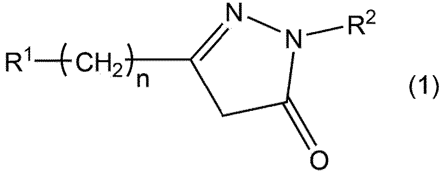
   wherein R¹ is an electron withdrawing group selected from the group consisting of one or more selected from the group consisting of a halogen atom, a nitro group, a cyano group, a haloalkyl group having 1 to 10 carbon atoms (one or more halogen atoms attached to the carbon atom at position 1), a perfluoropolyether group having 2 to 10 carbon atoms, a carboxy group,
   a substituted aryl group having 6-60 carbon atoms (wherein a benzene ring attached to the side of pyrazolone skeleton has at least one substituent, and said substituent is one or more group selected from a halogen atom, a nitro group, a cyano group, a haloalkyl group having 1 to 10 carbon atoms (one or more halogen atoms are attached to the carbon atom at position 1), perfluoropolyether group having 2 to 10 carbon atoms, a carboxy group, an acyl group having 2 to 61 carbon atoms, an alkoxycarbonyl group having 1 to 60 carbon atoms, an alkoxy group having 1 to 60 carbon atoms, and alkyl group having 1 to 60 carbon atoms; and when the substituent is the alkoxy group having 1 to 60 carbon atoms or the alkyl group having 1 to 60 carbon atoms, the position of the substituent is the meta-position of the benzene ring attached to the side of the pyrazolone skeleton), and the following formulas (2) to (7): 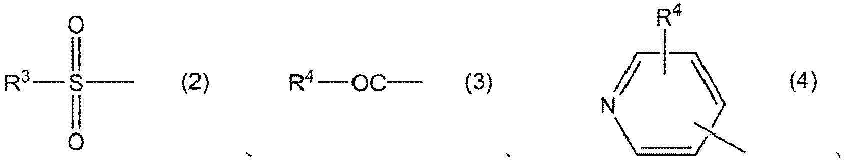 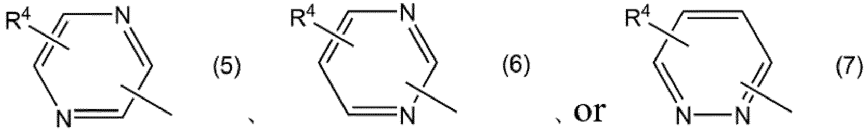 wherein
      R³ is a halogen atom, a nitro group, a cyano group, a haloalkyl group having 1 to 10 carbon atoms, a perfluoropolyether group having 2 to 10 carbon atoms, a carboxy group, an optionally substituted alkyl group having 1 to 60 carbon atoms,
      an optionally substituted alkenyl group having 1 to 60 carbon atoms, an optionally substituted alkynyl group having 1 to 60 carbon atoms, an optionally substituted aralkyl group having 1 to 60 carbon atoms, an optionally substituted aryl group having 6 to 60 carbon atoms, an optionally substituted alkoxy group having 1 to 60 carbon atoms, an optionally substituted alkenyloxy group having 1 to 60 carbon atoms, an optionally substituted alkynyloxy group having 1 to 60 carbon atoms, an optionally substituted aralkyloxy group having 1 to 60 carbon atoms, and/or an optionally substituted aryloxy group having 6 to 60 carbon atoms,
         and
      R⁴ is a halogen atom, a nitro group, a cyano group, a haloalkyl group having 1 to 10 carbon atoms, a perfluoropolyether group having 2 to 10 carbon atoms, a carboxy group, an optionally substituted alkyl group having 1 to 60 carbon atoms, an optionally substituted alkenyl group having 1 to 60 carbon atoms, an optionally substituted alkynyl group having 1 to 60 carbon atoms, an optionally substituted aralkyl group having 1 to 60 carbon atoms, an optionally substituted aryl group having 6 to 60 carbon atoms, an optionally substituted alkoxy group having 1 to 60 carbon atoms, an optionally substituted alkenyloxy group having 1 to 60 carbon atoms, an optionally substituted alkynyloxy group having 1 to 60 carbon atoms, an optionally substituted aralkyloxy group having 1 to 60 carbon atoms, an optionally substituted aryloxy group having 6 to 60 carbon atoms, an optionally substituted alkyl carbonyl group having 1 to 60 carbon atoms, an optionally substituted alkenyl carbonyl group having 1 to 60 carbon atoms, an optionally substituted alkynyl carbonyl group having 1 to 60 carbon atoms, an optionally substituted alalkyl carbonyl group having 1 to 60 carbon atoms, an optionally substituted arylcarbonyl group having 6 to 60 carbon atoms, an optionally substituted alkoxycarbonyl group having 1 to 60 carbon atoms, an optionally substituted alkenyloxycarbonyl group having 1 to 60 carbon atoms, an optionally substituted alkynyloxycarbonyl group having 1 to 60 carbon atoms, an optionally substituted aralkyloxycarbonyl group having 1 to 60 carbon atoms, and/or an optionally substituted aryloxycarbonyl group having 6 to 60 carbon atoms,
      and R⁴ is 0 to 4 in the formula (4), and R⁴ is 0 to 3 in the formulas (5) to (7), and R² is a hydrocarbon group having 1 to 1000 carbon atoms, which may contain a heteroatom, and n is an integer of 0 to 2,
[2] a pyrazolonating agent for formyl group-containing compound, comprising the pyrazolone compound of the item [1],
[3] a pyrazolonation method for formyl group-containing compound, characterized in that a formyl group-containing compound is brought into contact with the pyrazolone compound of the item [1],
[4] a method for stabilizing a pyrazolonated compound, characterized in that a formyl group-containing compound is brought into contact with the pyrazolone compound of the item [1],
[5] a synthesis method for pyrazolonated compound, characterized in that a formyl group-containing compound is brought into contact with the pyrazolone compound of the item [1],
[6] a pyrazolonated compound obtained by the synthesis method of the item [5].

### ADVANTAGEOUS EFFECTS OF INVENTION

According to the pyrazolone compound or the pyrazolonating agent of the present invention, a pyrazolonated compound exhibiting high stability can be obtained.

### BRIEF DESCRIPTION OF THE DRAWINGS

FIG. 1 is a graph showing that stabilities of pyrazolonated compounds (compounds 5 to 10) manufactured by using the pyrazolone compounds of the present invention, are examined.
FIG. 2 is a graph showing that stabilities of pyrazolonated compounds (compounds 5,and 28 to 31) manufactured by using the pyrazolone compounds of the present invention, are examined.
FIG. 3 is a graph showing that stabilities of pyrazolonated compounds (compounds 5,and 32 to 36) manufactured by using the pyrazolone compounds of the present invention, are examined.
FIG. 4 is a graph showing that stabilities of pyrazolonated compounds (compounds 5,and 37 to 41) manufactured by using the pyrazolone compounds of the present invention, are examined.
FIG. 5 is a graph showing that stabilities of pyrazolonated compounds (compounds 5,and 42 to 45) manufactured by using the pyrazolone compounds of the present invention, are examined.

### DESCRIPTION OF EMBODIMENTS

The pyrazolone compound of the present invention is a compound represented by the following formula (1):

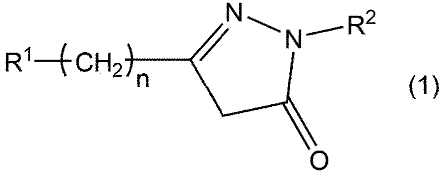

R¹ is an electron withdrawing group selected from the group consisting of one or more selected from the group consisting of a halogen atom, a nitro group, a cyano group, a haloalkyl group having 1 to 10 carbon atoms (one or more halogen atoms attached to the carbon atom at position 1), a perfluoropolyether group having 2 to 10 carbon atoms, a carboxy group, a substituted aryl group having 6-60 carbon atoms (wherein a benzene ring attached to the side of pyrazolone skeleton has at least one substituent, and said substituent is one or more group selected from a halogen atom, a nitro group, a cyano group, a haloalkyl group having 1 to 10 carbon atoms (one or more halogen atoms are attached to the carbon atom at position 1), perfluoropolyether group having 2 to 10 carbon atoms, a carboxy group, an acyl group having 2 to 61 carbon atoms, an alkoxycarbonyl group having 1 to 60 carbon atoms, an alkoxy group having 1 to 60 carbon atoms, and alkyl group having 1 to 60 carbon atoms; and when the substituent is the alkoxy group having 1 to 60 carbon atoms or the alkyl group having 1 to 60 carbon atoms, the position of the substituent is the meta-position of the benzene ring attached to the side of the pyrazolone skeleton), and the following formulas (2) to (7): 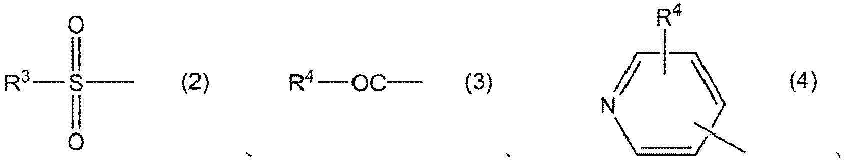 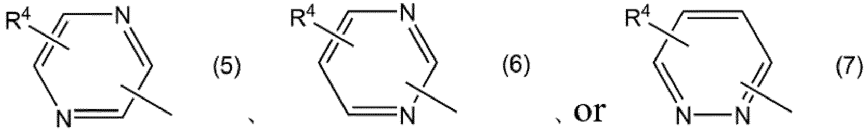 wherein
R³ is a halogen atom, a nitro group, a cyano group, a haloalkyl group having 1 to 10 carbon atoms, a perfluoropolyether group having 2 to 10 carbon atoms, a carboxy group, an optionally substituted alkyl group having 1 to 60 carbon atoms, an optionally substituted alkenyl group having 1 to 60 carbon atoms, an optionally substituted alkynyl group having 1 to 60 carbon atoms, an optionally substituted aralkyl group having 1 to 60 carbon atoms, an optionally substituted aryl group having 6 to 60 carbon atoms, an optionally substituted alkoxy group having 1 to 60 carbon atoms, an optionally substituted alkenyloxy group having 1 to 60 carbon atoms, an optionally substituted alkynyloxy group having 1 to 60 carbon atoms, an optionally substituted aralkyloxy group having 1 to 60 carbon atoms, and/or an optionally substituted aryloxy group having 6 to 60 carbon atoms, and
R⁴ is a halogen atom, a nitro group, a cyano group, a haloalkyl group having 1 to 10 carbon atoms, a perfluoropolyether group having 2 to 10 carbon atoms, a carboxy group, an optionally substituted alkyl group having 1 to 60 carbon atoms, an optionally substituted alkenyl group having 1 to 60 carbon atoms, an optionally substituted alkynyl group having 1 to 60 carbon atoms, an optionally substituted aralkyl group having 1 to 60 carbon atoms, an optionally substituted aryl group having 6 to 60 carbon atoms, an optionally substituted alkoxy group having 1 to 60 carbon atoms, an optionally substituted alkenyloxy group having 1 to 60 carbon atoms, an optionally substituted alkynyloxy group having 1 to 60 carbon atoms, an optionally substituted aralkyloxy group having 1 to 60 carbon atoms, an optionally substituted aryloxy group having 6 to 60 carbon atoms, an optionally substituted alkyl carbonyl group having 1 to 60 carbon atoms, an optionally substituted alkenyl carbonyl group having 1 to 60 carbon atoms, an optionally substituted alkynyl carbonyl group having 1 to 60 carbon atoms, an optionally substituted alalkyl carbonyl group having 1 to 60 carbon atoms, an optionally substituted arylcarbonyl group having 6 to 60 carbon atoms, an optionally substituted alkoxycarbonyl group having 1 to 60 carbon atoms, an optionally substituted alkenyloxycarbonyl group having 1 to 60 carbon atoms, an optionally substituted alkynyloxycarbonyl group having 1 to 60 carbon atoms, an optionally substituted aralkyloxycarbonyl group having 1 to 60 carbon atoms, and/or an optionally substituted aryloxycarbonyl group having 6 to 60 carbon atoms, and R⁴ is 0 to 4 in the formula (4), and R⁴ is 0 to 3 in the formulas (5) to (7). R² is a hydrocarbon group having 1 to 1000 carbon atoms, which may contain a heteroatom. Then, n is an integer of 0 to 2.

The pyrazolone compound of the present invention can be used for pyrazolonation of formyl group-containing compound.

The pyrazolone compound of the present invention may be a CH type of the above formula (1), but may be a structural isomer (OH type) represented by the following (8) or a structural isomer (NH type) represented by the following (9):

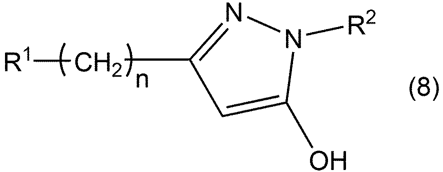

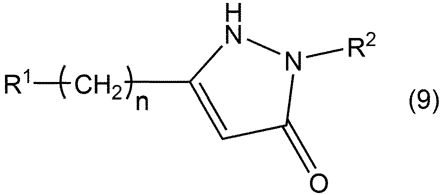

Since R¹ in the present invention is the electron withdrawing group, the pyrazolonated compound produced using the pyrazolone compound of the present invention is resistant to decomposition and can be a stable pyrazolonated compound.

As the halogen atoms, there may be mentioned a fluorine atom, a chlorine atom, a bromine atom, or an iodine atom. The pyrazolone compound wherein R¹ is halogen atom is not limited, but includes, for example,

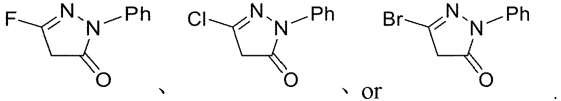

The nitro group is represented by -NO₂. The pyrazolone compound wherein R¹ is nitro group is not limited, but includes, for example,

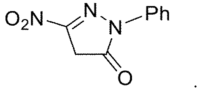

The cyano group is represented by -CN. The pyrazolone compound wherein R¹ is cyano group is not limited, but includes, for example,

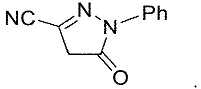

The haloalkyl group having 1 to 10 carbon atoms (one or more halogen atoms attached to the carbon atom at position 1) means an alkyl group in which one or more hydrogen atoms of the alkyl group having 1 to 10 carbon atoms are substituted with one or more halogen atoms such as fluorine atoms, chlorine atoms, bromine atoms, or iodine atoms. Preferably, it is a haloalkyl group having 1 to 8 carbon atoms, more preferably a haloalkyl group having 1 to 6 carbon atoms, and more preferably a haloalkyl group having 1 to 4 carbon atoms. In this specification, the alkyl group may be a linear alkyl group, a branched alkyl group, or a cyclic alkyl group. The larger the number of halogen atoms, the stronger the electron attraction, and thus the high number of halogen atoms is preferable. However, the presence of one or more halogen atoms (preferably two or more) attached to the carbon atom at position 1 allows the haloalkyl group to function as an electron-withdrawing group. In this specification, "carbon atom at position 1" in the haloalkyl group means a carbon atom bonded to -(CH₂)n- of R1. In the haloalkyl group having 2 to 10 carbon atoms, one or more halogen atoms (preferably two or more halogen atoms) are bonded to the carbon atom at position 2, although this is not limited. For example, haloalkyl groups include chloromethyl group, 2,2,2-trichloroethyl group, or 2,2,2-trifluoroethyl group.

A group in which all hydrogen atoms of an alkyl group are substituted with halogen atoms is referred to a perhaloalkyl group. For example, a perfluoroalkyl group means a group in which all hydrogen atoms of an alkyl group are substituted with fluorine atoms. Specifically, perfluoroalkyl groups include trifluoromethyl group, 2,2,2-trifluoro-1,1-dichloroethyl group, perfluoroethyl group, perfluoropropyl group, perfluoroisopropyl group, perfluorobutyl group, perfluoro sec-butyl group, perfluoro tert-butyl group, perfluoropentyl group, perfluorohexyl group, trichloromethyl group, tribromomethyl group, triiodomethyl group, or the like.

The pyrazolone compound in which R¹ is haloalkyl group having 1 to 10 carbon atoms (one or more halogen atoms attached to the carbon atom at position 1) is not limited, but includes, for example,

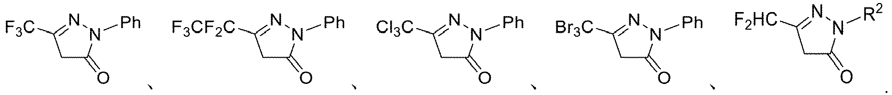

The haloalkyl group having 1 to 10 carbon atoms (one or more halogen atoms attached to the carbon atom at position 1) have a strong electron- withdrawing activity if one or more halogen atoms are bonded to the carbon atom at position 1, and thus the effect of the present invention can be obtained. Furthermore, a haloalkyl group in which two or more halogen atoms bonded to the carbon at position 1, or a haloalkyl group in which two or more halogen atoms bonded to the carbon at positions 1 and 2, have very strong electron- withdrawing activity, and thus the effect of the present invention can be obtained.

The perfluoropolyether group having 2 to 10 carbon atoms is a perfluoroalkyl group containing an ether group (-O-), and specifically, is not limited, but includes

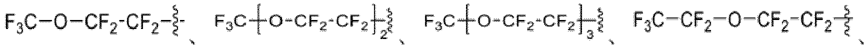

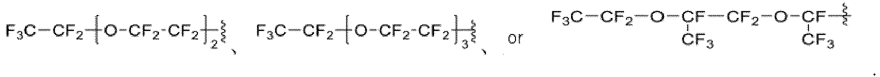

The carboxy group is represented by -COOH. The pyrazolone compound wherein R¹ is carboxy group is not limited, but includes, for example,

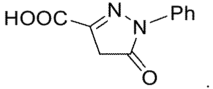

The substituted aryl group having 6-60 carbon atoms (wherein a benzene ring attached to the side of pyrazolone skeleton has at least one substituent, and said substituent is one or more group selected from a halogen atom, a nitro group, a cyano group, a haloalkyl group having 1 to 10 carbon atoms (one or more halogen atoms are attached to the carbon atom at position 1), perfluoropolyether group having 2 to 10 carbon atoms, a carboxy group, an acyl group having 2 to 61 carbon atoms, an alkoxycarbonyl group having 1 to 60 carbon atoms, an alkoxy group having 1 to 60 carbon atoms, and alkyl group having 1 to 60 carbon atoms; and when the substituent is the alkoxy group having 1 to 60 carbon atoms or the alkyl group having 1 to 60 carbon atoms, the position of the substituent is the meta-position of the benzene ring attached to the side of the pyrazolone skeleton), is basically a group in which the hydrogen atom of the benzene ring attached to the pyrazolone skeleton side is substituted with a substituent that is an electron-withdrawing group. At least one or more hydrogen atoms of the benzene ring bonded to the pyrazolone skeleton side are substituted, and it may have two substituents, three substituents, four substituents, or five substituents. In the case of having two or more substituents, the substituents may be the same or a combination of two or more of the above substituents. The position of the substituent of the electron-withdrawing group is not limited, but most preferably the para-position. The pyrazolone compound wherein R¹ is the substituted aryl group having 6-60 carbon atoms is not limited, but includes, for example,

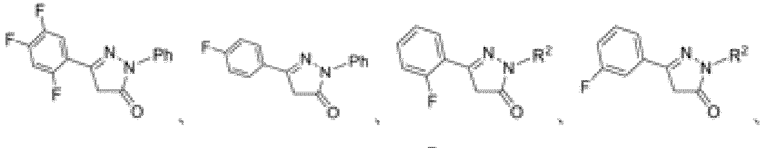

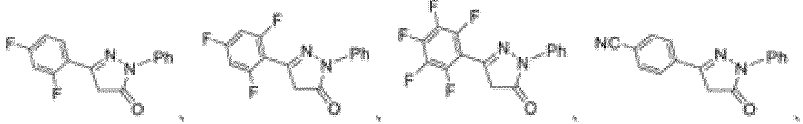

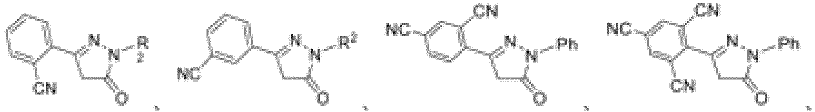

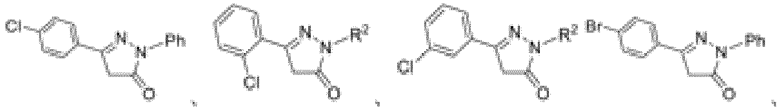

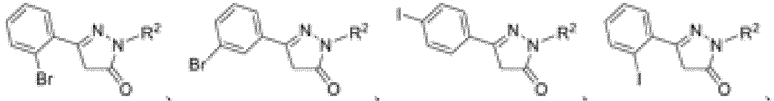

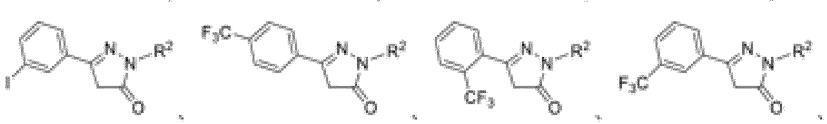

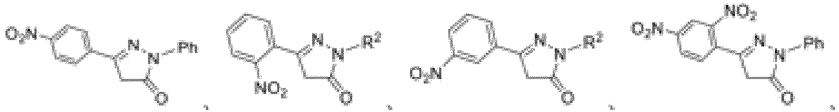

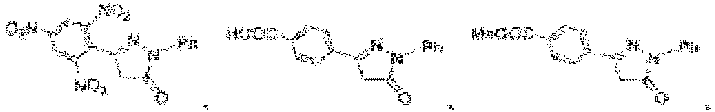

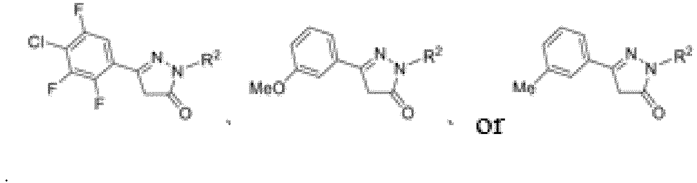

The acyl group having 2 to 61 carbon atoms is represented by R⁵-OC-, and R⁵ is hydrocarbon group. R⁵ is not limited, but includes an optionally substituted alkyl group having 1 to 60 carbon atoms, an optionally substituted alkenyl group having 1 to 60 carbon atoms, an optionally substituted alkynyl group having 1 to 60 carbon atoms, an optionally substituted aralkyl group having 1 to 60 carbon atoms, or an optionally substituted aryl group having 6 to 60 carbon atoms.

R¹ may be represented by the following formula (2): 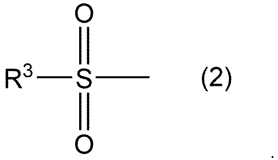 R³ is a halogen atom, a nitro group, a cyano group, a haloalkyl group having 1 to 10 carbon atoms, a perfluoropolyether group having 2 to 10 carbon atoms, a carboxy group, an optionally substituted alkyl group having 1 to 60 carbon atoms, an optionally substituted alkenyl group having 1 to 60 carbon atoms, an optionally substituted alkynyl group having 1 to 60 carbon atoms, an optionally substituted aralkyl group having 1 to 60 carbon atoms, an optionally substituted aryl group having 6 to 60 carbon atoms, an optionally substituted alkoxy group having 1 to 60 carbon atoms, an optionally substituted alkenyloxy group having 1 to 60 carbon atoms, an optionally substituted alkynyloxy group having 1 to 60 carbon atoms, an optionally substituted aralkyloxy group having 1 to 60 carbon atoms, and/or an optionally substituted aryloxy group having 6 to 60 carbon atoms.

When R³ is the haloalkyl group having 1 to 10 carbon atoms, it specifically includes a trifluoromethanesulfonyl group, a pentafluoroethanesulfonyl group, or the like. When R³ is the optionally substituted alkyl group having 1 to 60 carbon atoms, it specifically includes a methyl sulfonyl group (mesyl group), an ethyl sulfonyl group, a n-propyl sulfonyl group, an isopropyl sulfonyl group, a n-butyl sulfonyl group, an isobutyl sulfonyl group, a sec-butyl sulfonyl group, a tert-butyl sulfonyl group, or the like. When R³ is the optionally substituted aryl group having 6 to 60 carbon atoms, it specifically includes a phenyl sulfonyl group, a methyl phenyl sulfonyl group, a methyl para-tolyl sulfonyl group, a methyl meta-tolyl sulfonyl group, a methyl ortho-tolyl sulfonyl group, a 1-naphthyl sulfonyl group, a 2-naphthyl sulfonyl group, a 2-methyl phenyl sulfonyl group, or the like.

The group represented by the formula (2) functions as an electron-withdrawing group by means of the sulfonyl group close to the pyrazolone skeleton. Therefore, R³ which bonded thereto via the sulfonyl group close to the pyrazolone skeleton is considered to have no significant effect on the function as an electron-withdrawing group. Therefore, R³ can be an alkyl or an aryl group, which is not an electron-withdrawing group, a halogen atom or a nitro group, which is an electron-withdrawing group.

The pyrazolone compound wherein R¹ is the group represented by the formula (2), is not limited, but includes, for example,

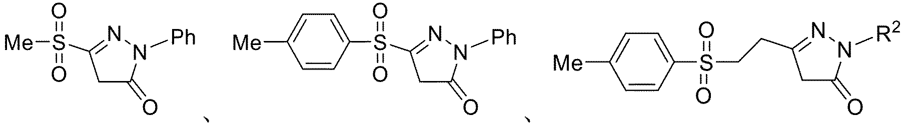

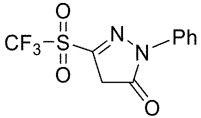

R¹ may be represented by the following formula (3):]

R⁴-OC- (3)

R⁴ is a halogen atom, a nitro group, a cyano group, a haloalkyl group having 1 to 10 carbon atoms, a perfluoropolyether group having 2 to 10 carbon atoms, a carboxy group, an optionally substituted alkyl group having 1 to 60 carbon atoms, an optionally substituted alkenyl group having 1 to 60 carbon atoms, an optionally substituted alkynyl group having 1 to 60 carbon atoms, an optionally substituted aralkyl group having 1 to 60 carbon atoms, an optionally substituted aryl group having 6 to 60 carbon atoms, an optionally substituted alkoxy group having 1 to 60 carbon atoms, an optionally substituted alkenyloxy group having 1 to 60 carbon atoms, an optionally substituted alkynyloxy group having 1 to 60 carbon atoms, an optionally substituted aralkyloxy group having 1 to 60 carbon atoms, an optionally substituted aryloxy group having 6 to 60 carbon atoms, an optionally substituted alkyl carbonyl group having 1 to 60 carbon atoms, an optionally substituted alkenyl carbonyl group having 1 to 60 carbon atoms, an optionally substituted alkynyl carbonyl group having 1 to 60 carbon atoms, an optionally substituted alalkyl carbonyl group having 1 to 60 carbon atoms, an optionally substituted arylcarbonyl group having 6 to 60 carbon atoms, an optionally substituted alkoxycarbonyl group having 1 to 60 carbon atoms, an optionally substituted alkenyloxycarbonyl group having 1 to 60 carbon atoms, an optionally substituted alkynyloxycarbonyl group having 1 to 60 carbon atoms, an optionally substituted aralkyloxycarbonyl group having 1 to 60 carbon atoms, and/or an optionally substituted aryloxycarbonyl group having 6 to 60 carbon atoms.

When R⁴ is the optionally substituted alkoxy group having 1 to 60 carbon atoms, it is an alkoxy group having 1 to 30 carbon atoms in one embodiment, an alkoxy group having 1 to 20 carbon atoms in one embodiment, an alkoxy group having 1 to 10 carbon atoms in one embodiment. Specifically, the group of formula (3) includes a methoxycarbonyl group, an ethoxycarbonyl group, a butoxycarbonyl group, or the like. The alkoxy group having 1 to 60 carbon atoms functions as an electron-withdrawing group by means of the ester moiety (or carbonyl moiety) close to the pyrazolone skeleton. Therefore, the groups which bonded thereto via the ester moiety close to the pyrazolone skeleton is considered to have no significant effect on the function as an electron-withdrawing group. Thus, for example, the alkoxy group having 60 carbon atoms can also function as an electron-withdrawing group. The pyrazolone compound wherein R¹ is the optionally substituted alkoxy group having 1 to 60 carbon atoms is not limited, but includes, for example,

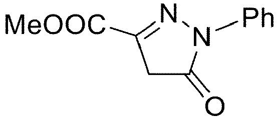

When R⁴ is the optionally substituted alkyl group having 1 to 60 carbon atoms, it is an alkyl group having 1 to 30 carbon atoms in one embodiment, an alkyl group having 1 to 20 carbon atoms in one embodiment, an alkyl group having 1 to 10 carbon atoms in one embodiment. Specifically, the group of formula (3) includes an acetyl group, a propionyl group, a butanoyl group, a 2-methylpropionyl group, a heptanoyl group, a 2-methylbutanoyl group, a 3-methylbutanoyl group, an octanoyl group, a decanoyl group, a dodecanoyl group, an octadecanoyl group, or the like. The alkyl group having 1 to 60 carbon atoms functions as an electron-withdrawing group by means of the carbonyl group moiety close to the pyrazolone skeleton. Therefore, the groups which bonded thereto via the carbonyl group close to the pyrazolone skeleton is considered to have no significant effect on the function as an electron-withdrawing group. Thus, the alkyl group having 60 carbon atoms can also function as an electron-withdrawing group. The pyrazolone compound wherein R¹ is the optionally substituted alkyl group having 1 to 60 carbon atoms is not limited, but includes, for example,

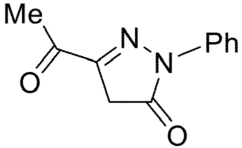

When R⁴ is the optionally substituted aryl group having 6 to 60 carbon atoms, it is an aryl group having 6 to 30 carbon atoms in one embodiment, an aryl group having 6 to 22 carbon atoms in one embodiment, an aryl group having 6 to 10 carbon atoms in one embodiment. Specifically, the group of formula (3) includes a benzoyl group, a methylbenzoyl group, an ethylbenzoyl group, a propylbenzoyl group, a butylbenzoyl group, a dimethylbenzoyl group, a 1-phthylcarbonyl group, a naphthylcarbonyl group, or the like. The aryl group having 6 to 60 carbon atoms functions as an electron-withdrawing group by means of the carbonyl group moiety close to the pyrazolone skeleton. Therefore, the aryl groups which bonded thereto via the carbonyl group close to the pyrazolone skeleton is considered to have no significant effect on the function as an electron-withdrawing group. Thus, the alkyl group having 60 carbon atoms can also function as an electron-withdrawing group. The pyrazolone compound wherein R¹ is the optionally substituted aryl group having 6 to 10 carbon atoms is not limited, but includes, for example,

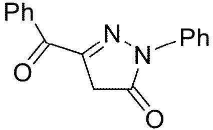

R¹ may be represented by the following formulas (4)-(7): 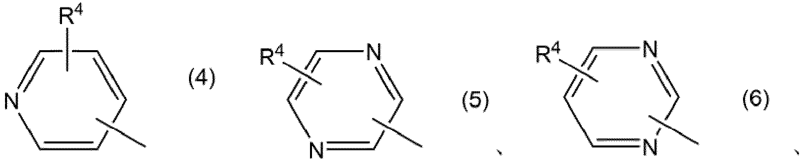 or

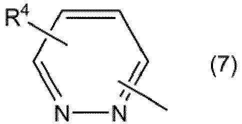

R⁴ is a halogen atom, a nitro group, a cyano group, a haloalkyl group having 1 to 10 carbon atoms, a perfluoropolyether group having 2 to 10 carbon atoms, a carboxy group, an optionally substituted alkyl group having 1 to 60 carbon atoms, an optionally substituted alkenyl group having 1 to 60 carbon atoms, an optionally substituted alkynyl group having 1 to 60 carbon atoms, an optionally substituted aralkyl group having 1 to 60 carbon atoms, an optionally substituted aryl group having 6 to 60 carbon atoms, an optionally substituted alkoxy group having 1 to 60 carbon atoms, an optionally substituted alkenyloxy group having 1 to 60 carbon atoms, an optionally substituted alkynyloxy group having 1 to 60 carbon atoms, an optionally substituted aralkyloxy group having 1 to 60 carbon atoms, an optionally substituted aryloxy group having 6 to 60 carbon atoms, an optionally substituted alkyl carbonyl group having 1 to 60 carbon atoms, an optionally substituted alkenyl carbonyl group having 1 to 60 carbon atoms, an optionally substituted alkynyl carbonyl group having 1 to 60 carbon atoms, an optionally substituted alalkyl carbonyl group having 1 to 60 carbon atoms, an optionally substituted arylcarbonyl group having 6 to 60 carbon atoms, an optionally substituted alkoxycarbonyl group having 1 to 60 carbon atoms, an optionally substituted alkenyloxycarbonyl group having 1 to 60 carbon atoms, an optionally substituted alkynyloxycarbonyl group having 1 to 60 carbon atoms, an optionally substituted aralkyloxycarbonyl group having 1 to 60 carbon atoms, and/or an optionally substituted aryloxycarbonyl group having 6 to 60 carbon atoms. R⁴ is 0 to 4 in the formula (4), and R⁴ is 0 to 3 in the formulas (5) to (7).

The group represented by the formula (4) is an optionally substituted pyridyl group, i.e. a residue in which one hydrogen atom is removed from pyridine (C₅H₅N). The position on the pyridine ring removing one hydrogen atom can be any carbon atom in the ortho, meta or para position with respect to the nitrogen atom. That is, pyridyl group includes 2-pyridyl group, 3-pyridyl group, or 4-pyridyl group. R⁴ is substituted with one or more hydrogen atoms of a pyridyl group, and may have one, two, three, or four substituents. When R⁴ is 0, it means a pyridyl group without substituents. When it has two or more substituents, the substituents may be the same or a combination of two or more of the above substituents. The pyrazolone compound wherein R¹ is the group represented by the formula (4) is not limited, but includes, for example, 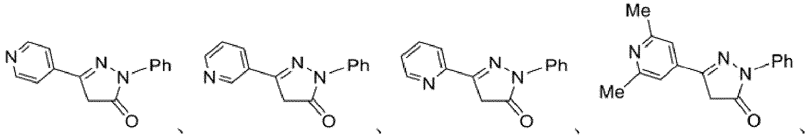 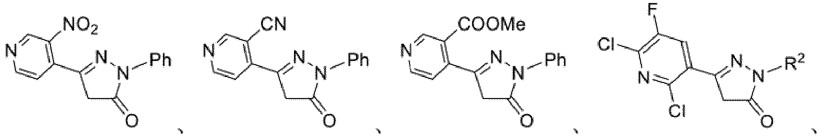 or

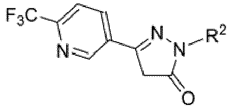

The group represented by the formula (5) to (7) is an optionally substituted diazine group, i.e. a residue in which one hydrogen atom is removed from diazine (C₄H₄N₂). Diazine includes three isomers, namely pyrazine, pyrimidine, or pyridazine, depending on the position of the nitrogen. One hydrogen atom may be removed at any position of carbon atom. R⁴ is substituted with one or more hydrogen atoms of a diazine group, and may have one, two, or three, substituents. When R⁴ is 0, it means a pyrazine, pyrimidine, or pyridazine group without substituents. When it has two or more substituents, the substituents may be the same or a combination of two or more of the above substituents. The pyrazolone compound wherein R¹ is the group represented by the formulas (5) to (7) is not limited, but includes, for example,

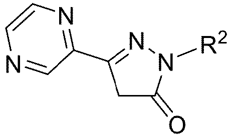

The halogen atom, nitro group, cyano group, haloalkyl group having 1 to 10 carbon atoms, or carboxy group in R³ and R⁴ of the formulas (2) to (7), is the same as that described in R¹. The number of carbon atoms in an optionally substituted alkyl group having 1 to 60 carbon atoms, an optionally substituted alkenyl group having 1 to 60 carbon atoms, an optionally substituted alkynyl group having 1 to 60 carbon atoms, an optionally substituted aralkyl group having 1 to 60 carbon atoms, an optionally substituted alkoxy group having 1 to 60 carbon atoms, an optionally substituted alkenyloxy group having 1 to 60 carbon atoms, an optionally substituted alkynyloxy group having 1 to 60 carbon atoms, an optionally substituted aralkyloxy group having 1 to 60 carbon atoms, an optionally substituted alkyl carbonyl group having 1 to 60 carbon atoms, an optionally substituted alkenyl carbonyl group having 1 to 60 carbon atoms, an optionally substituted alkynyl carbonyl group having 1 to 60 carbon atoms, an optionally substituted alalkyl carbonyl group having 1 to 60 carbon atoms, an optionally substituted alkoxycarbonyl group having 1 to 60 carbon atoms, an optionally substituted alkenyloxycarbonyl group having 1 to 60 carbon atoms, an optionally substituted alkynyloxycarbonyl group having 1 to 60 carbon atoms, an optionally substituted aralkyloxycarbonyl group having 1 to 60 carbon atoms, and/or an optionally substituted aryloxycarbonyl group having 6 to 60 carbon atoms, is 1 to 30 of carbon number in one embodiment, 1 to 20 of carbon number in one embodiment, 1 to 10 of carbon number in one embodiment, In addition, the number of carbon atoms in an optionally substituted aryl group having 6 to 60 carbon atoms, an optionally substituted aryloxy group having 6 to 60 carbon atoms, or an optionally substituted arylcarbonyl group having 6 to 60 carbon atoms, is 6 to 30 of carbon number in one embodiment, 6 to 20 of carbon number in one embodiment, 6 to 10 of carbon number in one embodiment,

The substituent of hydrogen atom of optionally substituted alkyl group having 1 to 60 carbon atoms and the like, is not particularly limited, as long as the effect of the present invention can be achieved. However, there may be mentioned a halogen atom, a nitro group, a cyano group, a haloalkyl group having 1 to 10 carbon atoms, a carboxy group, an alkyl group having 1 to 10 carbon atoms, an alkenyl group having 1 to 10 carbon atoms, an alkynyl group having 1 to 10 carbon atoms, an aralkyl group having 1 to 10 carbon atoms, an aryl group having 6 to 10 carbon atoms, an alkoxy group having 1 to 10 carbon atoms, an alkenyloxy group having 1 to 10 carbon atoms, an alkynyloxy group having 1 to 10 carbon atoms, an aralkyloxy group having 1 to 10 carbon atoms, an aryloxy group having 6 to 10 carbon atoms, an alkyl carbonyl group having 1 to 10 carbon atoms, an alkenyl carbonyl group having 1 to 10 carbon atoms, an alkynyl carbonyl group having 1 to 10 carbon atoms, an alalkyl carbonyl group having 1 to 10 carbon atoms, an arylcarbonyl group having 6 to 10 carbon atoms, an alkoxycarbonyl group having 1 to 10 carbon atoms, an alkenyloxycarbonyl group having 1 to 10 carbon atoms, an alkynyloxycarbonyl group having 1 to 10 carbon atoms, an aralkyloxycarbonyl group having 1 to 10 carbon atoms, and/or an aryloxycarbonyl group having 6 to 10 carbon atoms. The carbon number of 1 to 10 is preferably a carbon number of 1 to 6, preferably a carbon number of 1 to 4. The carbon number of 6 to 10 is preferably a carbon number of 6.

R² in the pyrazolone compounds of the present invention is not particularly limited, as long as the effect of the present invention can be achieved, but includes a hydrocarbon group having 1 to 1000 carbon atoms in one embodiment, a hydrocarbon group having 1 to 200 carbon atoms in one embodiment, a hydrocarbon group having 1 to 100 carbon atoms in one embodiment, which may contain a heteroatom. The heteroatom is not particularly limited, but includes nitrogen atom (N), oxygen atom (O), sulfur atom (S), phosphorus atom (P), chlorine atom (Cl), iodine atom (I), bromine atom (Br), fluorine atom (F), or silicon atom (Si) The group of R² may contain one or more of the heteroatoms, and may contain two or more heteroatoms of different types. R² does not affect the stability of pyrazolonated compounds prepared with the pyrazolone compounds of the present invention, as described below. Therefore, R² is not basically limited. The hydrocarbon group include, for example, a saturated or unsaturated chain hydrocarbon group, an alicyclic hydrocarbon group, an aromatic group, or a polyethylene glycol group. More specifically, the hydrocarbon group having 1 to 1000 carbon atoms, which may contain a heteroatom, includes, for example, polymers including polyethylene glycol, polyols, amino acids and their derivatives, peptides, proteins, nucleic acids, sugars, their derivatives, and the like.

n is an integer of 0 to 2. The group -(CH₂)n- is a bonding group between the pyrazolone skeleton and R¹. If the distance between R¹, which is the electron withdrawing group, and the pyrazolone skeleton is a certain distance or less, it is considered that the effect of the present invention can be achieved. That is, the group -(CH₂)n- is preferably shorter, therefore, n is preferably an integer of 0 or 1, more preferably 0. However, in the electron- withdrawing groups with very strong electron-withdrawing activity (such as halogen atom, nitro group, cyano group, haloalkyl group having 1 to 10 carbon atoms (one or more halogen atoms are attached to the carbon atom at position 1), and perfluoropolyether group having 2 to 10 carbon atoms), the invention of n=2 sufficiently obtain the effect of the present invention. For electron-withdrawing groups with strong electron- withdrawing activity (such as carboxy groups and substituted aryl groups having 6 to 60 carbon atoms), the effect of the present invention can be obtained with n=2, but preferably with n=1 or n=0.

The pyrazolone compound of the present invention may be a form of salt acceptable as the pyrazolonating agent, as described below. That is, it may form an acid addition salt or a salt with base.

Specific examples of the salt with the base include a salt with an inorganic base, an organic base, or a metallic alkoxide. The salts can be prepared by mixing the pyrazolone compound of the present invention with an inorganic base, an organic base, or a metallic alkoxide.

As the inorganic bases that can form salts, there may be mentioned a hydroxide, carbonate, hydrogen carbonate, acetate, or hydride of alkali metals (such as lithium, sodium, potassium, or the like); a hydroxide, hydride, or the like of alkaline earth metals (such as magnesium, calcium, or barium). As the organic bases that can form salts, there may be mentioned dimethylamine, triethylamine, piperazine, pyrrolidine, piperidine, 2-phenylethylamine, benzylamine, ethanolamine, diethanolamine, pyridine, collagen, or the like. Further, as the metallic alkoxide, there may be mentioned sodium methoxide, potassium tert-butoxide, magnesium methoxide, or the like.

As the specific acid addition salts, there may be mentioned salts with inorganic acids or organic acids. Acid addition salts can be formed by mixing the compound [1] of the present invention with inorganic acids or organic acids.

The inorganic acids include hydrochloric acid, hydrobromic acid, hydroiodic acid, sulfuric acid, nitric acid, phosphoric acid, or the like. The organic acids include formic acid, acetic acid, propionic acid, oxalic acid, malonic acid, succinic acid, fumaric acid, maleic acid, lactic acid, malic acid, mandelic acid, tartaric acid, dibenzoyl tartraric acid, ditoluoyl artraric acid, citric acid, methane sulfonic acid, ethane sulfonic acid, benzene sulfonic acid, p-toluene sulfonic acid, aspartic acid, glutamic acid, benzoic acid, camphor sulfonic acid, ethenesulfonic acid, gluconic acid, hydrobromic acid, isethionic acid, mucic acid, pamoic acid, pantothenic acid, or the like.

The synthesis method of the pyrazolone compound of the present invention is not particularly limited, and any method known in this field can be used without restriction.

The pyrazolonating agent of the present invention contains the pyrazolone compound of the present invention and can pyrazolonate the formyl group of formyl group-containing compound. Specifically, as shown below, the pyrazolonated compound wherein two molecules of the pyrazolone compound are bound, can be obtained by reacting the formyl group-containing compound such as a protein, peptide, or sugar having the formyl group with a pyrazolone compound of the present invention.

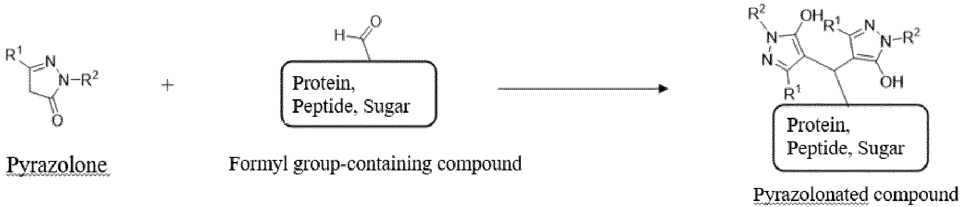

The pyrazolonating agent of the present invention may contain a carrier (such as water or buffer solution), a filler, a diluent, a preservative, a stabilizer, an antiseptic, or an antioxidant, as ingredients other than the pyrazolone compound.

The formyl group-containing compound is not particularly limited as long as it contains the formyl group. For example, there may be mentioned formyl group-containing antibodies, drugs, peptides, amino acids and their derivatives, peptides, proteins, nucleic acids, sugars and their derivatives, or sugars.

In the pyrazolonation method for formyl group-containing compound of the present invention, the pyrazolone compound of the present invention is brought into contact with the formyl group-containing compound.

Specifically, the reaction is usually carried out in a solvent, and the reaction temperature is usually 0°C to 100°C, preferably 10°C to 50°C from the viewpoint of reaction rate and reaction efficiency.

The reaction time depends on the amount and type of substrate and solvent, as well as the reaction temperature, and is usually from 5 min to 180 hours, the reaction time is preferably from 30 min to 48 hous from the viewpoint of reaction rate and reaction efficiency.

The theoretical amount of pyrazolone compound is 2 moles per mole of formyl group-containing compound, and can be changed as desired depending on the reaction conditions. Usually, the amount of pyrazolone compound is 1 to 20 moles per mole of formyl group-containing compound, preferably 1 to 10 moles.

The reaction pressure is not particularly limited, and can be pressurized, atmospheric pressure, or reduced pressure, and is usually 0.01 to 10 MPa (hereafter, pressure is expressed in absolute pressure), and preferably 0.1 to 1 MPa.

In the method for stabilizing a pyrazolonated compound, the pyrazolone compound is brought into contact with the formyl group-containing compound. The reaction conditions for the reaction of the pyrazolone compound with the formyl group-containing compound can be used those as same as the pyrazolonation method for formyl group-containing compound. The resulting pyrazolonated compound is not easily decomposed and is stable.

In the synthesis method for pyrazolonated compound, the pyrazolone compound is brought into contact with the formyl group-containing compound. The reaction conditions for the reaction of the pyrazolone compound with the formyl group-containing compound can be used those as same as the pyrazolonation method for formyl group-containing compound.

The pyrazolonated compounds of the present invention can be prepared by the synthesis method for pyrazolonated compound of the present invention

### <<Functions>>

The mechanism wherein the pyrazolone compounds obtained in the present invention are stable and resistant to decomposition has not been analyzed in detail, but may be presumed to be as follows. However, the present invention is not limited by the following presumption

The pyrazolone compounds are isomeric mixtures of CH, OH, and NH isomers, as described above. Therefore, the pyrazolonated compounds obtained from the pyrazolone compounds are also isomeric mixtures of CH, OH, and NH isomers. According to the analysis of the present inventors, it was found that among the isomeric mixtures of pyrazolonated compounds, the CH isomer is the most susceptible to decomposition. When R¹ is an electron-withdrawing group, the pyrazolone ring is considered to have a stable cyclic resonance structure, the OH form. In other words, when R¹ is an electron-withdrawing group, the obtained pyrazolonated compound has a stable OH structure, and thus the stability of the pyrazolonated compound is high, and it is presumed that the pyrazolonated compound does not decompose.

On the other hand, R² is not involved in the conjugation system in the formation of the above cyclic resonance structure. Thus, it is considered that R² is not affected if R¹ is the electron-withdrawing group. Therefore, it is presumed that the structure of R² does not affect the stability of pyrazolonated compounds in any way, and the structure of R² is not limited.

### EXAMPLES

The present invention now will be further illustrated by, but is by no means limited to, the following Examples.

The structure of pyrazolone exists in equilibrium with tautomers as shown in Formula (1), (8), or (9) in the present specification. However in this example, only the CH form of Formula (1) is described.

### <<Example 1-4>>

In these examples, pyrazolone derivatives were prepared.

### (Preparation of Compound 1) (Example 1)

Ethyl 4,4,5,5,5-pentafluoro-3-oxovalerate (0.38 mL, 2.2 mmol) was dissolved in acetic acid (5mL), and phenylhydrazine (0.26 mL, 2.6 mmol) was added thereto. After stirring at 90°C for 23 hours, the reaction solution was extracted with ethyl acetate. The organic phase was washed with water, saturated sodium bicarbonate solution, and saturated saline in this order, and dried over anhydrous magnesium sulfate. After filtration and concentration of the extract, the residue was purified by silica gel column chromatography (hexane:ethyl acetate = 10:1-2:1) to obtain 428.3 mg (71% yield) of compound 1. 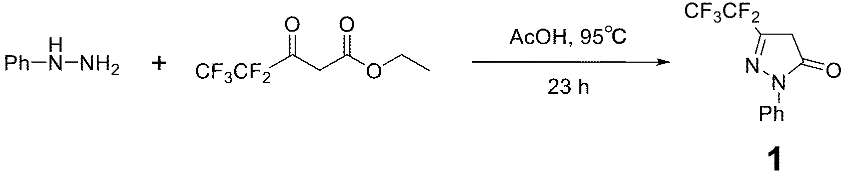 Compound 1 ¹H-NMR(600MHz, DMSO-d₆):δ=12.53(brs, 1H), 7.74-7.67(m, 2H), 7.56-7.49(m, 2H), 7.43-7.36(m, 1H), 5.93(s, 1H).

### (Preparation of Compound 2) (Example 2)

Ethyl (2,4,5-trifluorobenzoyl)acetate (501.7 mg, 2.04 mmol) was dissolved in acetic acid (5 mL), and phenylhydrazine (0.22 mL, 2.2 mmol) was added. After stirring at 85°C for 24 hours, the reaction solution was extracted with ethyl acetate. The organic phase was washed with water, saturated sodium bicarbonate solution, and saturated saline in this order, and dried over anhydrous magnesium sulfate. After filtration and concentration of the extract, the residue was purified by silica gel column chromatography (hexane:ethyl acetate=6:1-3.5:1) to obtain 482.3 mg (81% yield) of compound 2. 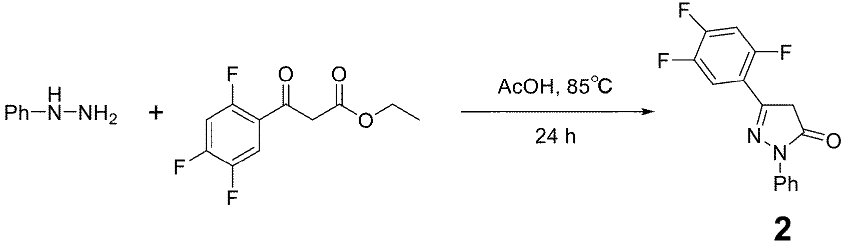 Compound 2 ¹H-NMR(600MHz, DMSO-d₆):δ=12.01(m, 1H), 8.02-7.92(m, 1H), 7.87-7.78(m, 2H), 7.71-7.61(m, 1H), 7.54-7.46(m, 2H), 7.37-7.28(m, 1H), 6.00-5.94(m, 1H).

### (Preparation of Compound 3) (Example 3)

Ethyl 2-(4-nitrobenzoyl) acetate (501.1 mg, 2.11 mmol) was dissolved in ethanol (5 mL) and phenylhydrazine (220 µL, 2.24 mmol) was added. After stirring the mixture under heating at reflux for 22 hours, the solvent was removed under reduced pressure. The residue was extracted with ethyl acetate. The organic phase was washed with saturated sodium bicarbonate solution and saturated saline in this order, and dried over anhydrous magnesium sulfate. The extract was filtered and concentrated, and the residue was recrystallized with ethanol to obtain 269.9 mg (45% yield) of compound 3. 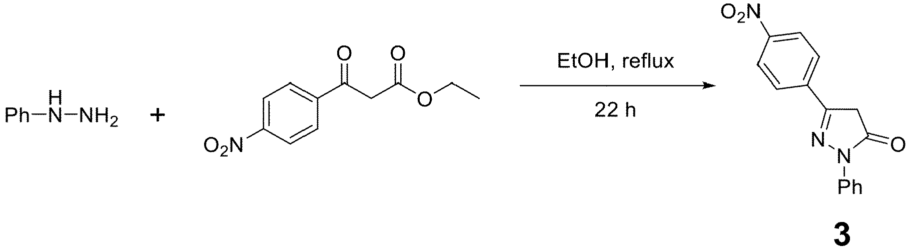 Compound 3 ¹H-NMR(600MHz, DMSO-d₆):δ=12.11(brs, 1H), 8.31-8.25(m, 2H), 8.15-8.08(m, 2H), 7.85-7.80(m, 2H), 7.55-7.47(m, 2H), 7.37-7.31(m, 1H), 6.22(s, 1H).

### (Preparation of Compound 4) (Example 4)

Ethyl 3-oxo-3-(4-pyridyl)propionate (414.8 mg, 2.15 mmol) was dissolved in acetic acid (5 mL) and phenylhydrazine (240 µL, 2.44 mmol) was added. After stirring at 85°C for 23 hours, the reaction solution was extracted with ethyl acetate. The organic phase was washed with water, saturated sodium bicarbonate solution, and saturated saline in this order, and dried over anhydrous magnesium sulfate. After filtration and concentration of the extract, the residue was purified by silica gel column chromatography (chloroform:methanol = 30:1-20:1) to obtain 174.6 mg (34% yield) of compound 4. 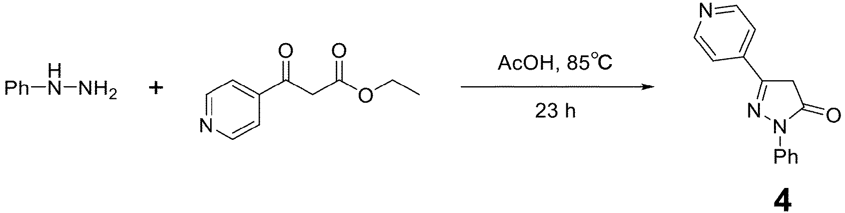 Compound 4 ¹H-NMR(600MHz, DMSO-d₆):δ=12.06(brs, 1H), 8.63-8.56(m, 2H), 7.85-7.76(m, 4H), 7.55-7.46(m, 2H), 7.36-7.29(m, 1H), 6.18(s, 1H).

### <<Comparative Example 1 and Examples 5 to 9>>

In these examples, pyrazolone derivatives were introduced into galactopyranosides.

### (Preparation of Compound 5) (Comparative Example 1)

Water (3.7 mL) and 3-methyl-1-phenyl-5-pyrazolone (100 mM DMF solution, 1.5 mL, 150 µmol) were added to 4-nitrophenyl β-D-galacto-hexodialdo-1,5-pyranoside (11.2 mg, 37.4 µmol), in this order. After stirring at room temperature for 1 hour, the reaction solution was concentrated under reduced pressure. The residue was purified by silica gel column chromatography (chloroform:methanol = 30:1 to 10:1) to obtain 18.4 mg (78% yield) of compound 5. 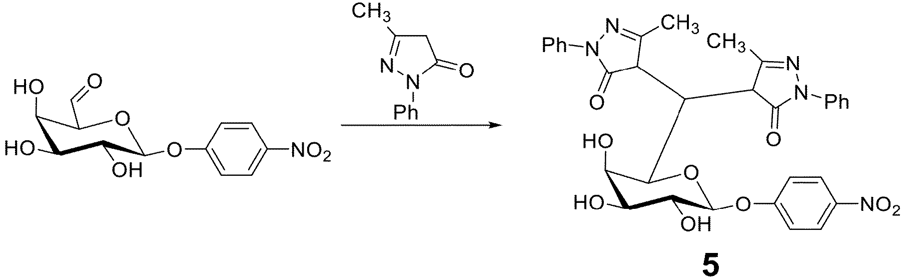 Compound 5 MALDI-TOF MS(AXIMA-TOF2) m/z[M+H]⁺ calcd for C₃₂H₃₂N₅O₉, 630.22, found 630.25.

### (Preparation of Compound 6) (Example 5)

Water (4.6 mL), 3-trifluoromethyl-1-phenyl-5-pyrazolone (100 mM DMF solution, 1.9 mL, 190 µmol), and DMF (1.5 mL) were added to 4-nitrophenyl β-D-galacto-hexodialdo-1,5-pyranoside (13.7 mg, 45.7 µmol), in this order. After stirring at room temperature for 22 hours, the reaction solution was concentrated under reduced pressure. The residue was purified by solid-phase extraction column (Sep-Pak C18, methanol:H2O=1:4-4:1, Waters), and high-performance liquid chromatography (ODS column 250×10mm, 5um, eluentA:0.1% aq.TFA, B:CH₃CN(0.1%TFA), B 55%-70% (30 min), 30 °C, UV240 nm) in this order, to obtain 0.7 mg of compound 6. 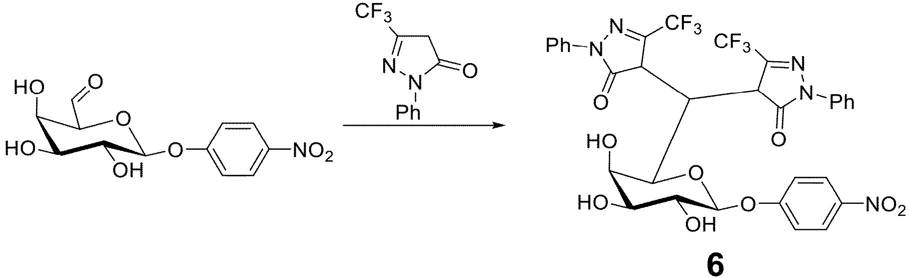 Compound 6 MALDI-TOF MS(AXIMA-TOF2) m/z[M+H]⁺ calcd for C₃₂H₂₆F₆N₅O₉, 738.16, found 737.97.

### (Preparation of Compound 7) (Example 6)

Water (4.3 mL), compound 1 (100 mM DMF solution, 1.8 mL, 180 µmol), and DMF (1.5 mL) were added to 4-nitrophenyl β-D-galacto-hexodialdo-1,5-pyranoside (12.8 mg, 42.8 µmol), in this order. After stirring at room temperature for 24 hours, the reaction solution was concentrated under reduced pressure. The residue was purified by solid-phase extraction column (Sep-Pak C18, methanol:H2O=1:4-3:2, Waters), and high-performance liquid chromatography (ODS column 250×10mm, 5um, eluent A: 0.1% aq.TFA, B: CH3CN(0.1% TFA), B 55%-70% (30 min), 30 °C, UV 240 nm), in this order, to obtain. 2.0 mg of compound 7. 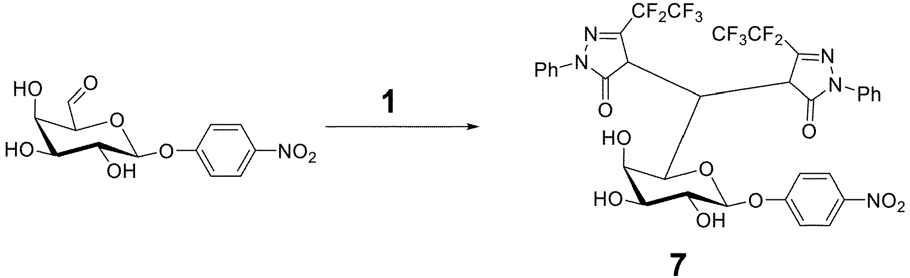 Compound 7 MALDI-TOFMS(AXIMA-TOF2) m/z[M+Na]⁺ calcd for C₃₄H₂₅F₁₀N₅O₉Na, 860.14, found 860.24.

### (Preparation of Compound 8) (Example 7)

Water (4.6 mL), compound 2 (100 mM DMF solution, 1.9 mL, 190 µmol), and DMF (4.5 mL) were added to 4-nitrophenyl β-D-galacto-hexodialdo-1,5-pyranoside (13.9 mg, 46.5 µmol), in this order. After stirring at room temperature for 24 hours, the reaction solution was concentrated under reduced pressure. The residue was purified by silica gel column chromatography (chloroform:methanol = 25:1-8:1) to obtain 32.6 mg (82% yield) of compound 8. 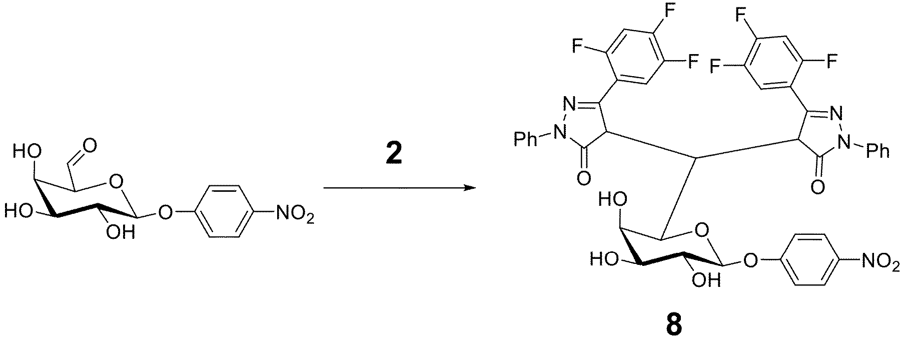 Compound 8 ¹H-NMR(600MHz, CD₃OD):δ=7.91-7.86(m, 2H), 7.82-7.77(m, 2H), 7.75-7.63(m, 3H), 7.59-7.53(m, 2H), 7.52-7.46(m, 2H), 7.44-7.39(m, 1H), 7.38-7.33(m, 1H), 7.17-7.01(m, 3H), 7.00-6.95(m, 2H), 5.06(d, *J*=7.7Hz, 1H), 4.95-4.87(m, 1H, overlapped with water signal), 3.99(d, *J*=10.8Hz, 1H), 3.90(dd, *J*=7.8, 9.5Hz, 1H), 3.81(d, *J*=3.2Hz, 1H), 3.71(dd, *J*=3.3, 9.6Hz, 1H).

### (Preparation of Compound 9) (Example 8)

Water (4.5 mL), compound 3 (100 mM DMF solution, 1.8 mL, 180 µmol), and DMF (4.5 mL) were added to 4-nitrophenyl β-D-galacto-hexodialdo-1,5-pyranoside (13.4 mg, 44.8 µmol), in this order. After stirring at room temperature for 22 hours, the reaction solution was concentrated under reduced pressure. The residue was purified by silica gel column chromatography (chloroform:methanol = 25:1-6:1) to obtain 31.9 mg (84% yield) of compound 9. 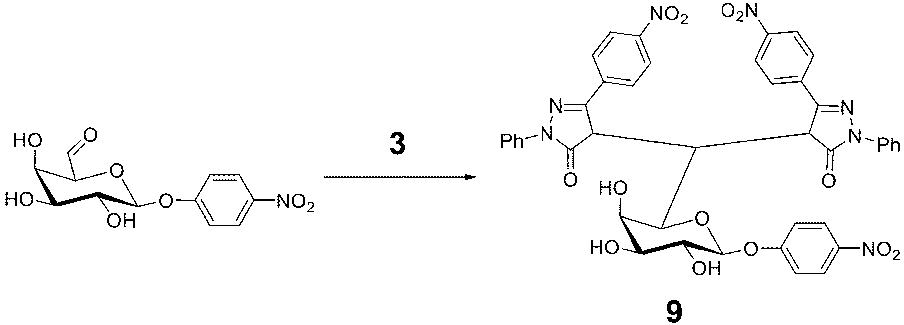 Compound 9 ¹H-NMR(600MHz, CD₃OD):δ=8.03-7.97(m, 2H), 7.89-7.82(m, 6H), 7.80-7.75(m, 2H), 7.65-7.61(m, 2H), 7.58-7.51(m, 4H), 7.50-7.44(m, 2H), 7.42-7.37(m, 1H), 7.35-7.30(m, 1H), 6.99-6.95(m, 2H), 5.09-5.03(m, 2H), 4.46(d, *J*=10.8 Hz, 1H),3.91-3.85(m, 2H), 3.74(dd, *J*=3.3, 9.6 Hz, 1H).

### (Preparation of Compound 10) (Example 9)

Water (4.9 mL), compound 4 (100 mM DMF solution, 1.96 mL, 196 µmol), and DMF (4.5 mL) were added to 4-nitrophenyl β-D-galacto-hexodialdo-1,5-pyranoside (14.6 mg, 48.8 µmol), in this order. After stirring at room temperature for 24 hours, the reaction solution was concentrated under reduced pressure. The residue was purified by silica gel column chromatography (chloroform:methanol=10:1-4:1-chloroform:methanol:water=12:8:1) to obtain 29.5 mg (80% yield) of compound 10. 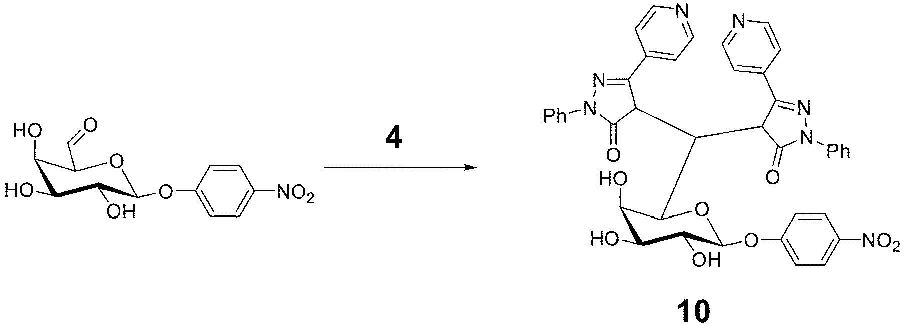
Compound 10 ¹H-NMR(600MHz, CD₃OD):δ=8.31(d, *J*=5.8Hz, 2H), 8.23(d, *J*=5.5Hz, 2H), 7.96(d, *J*=7.9Hz, 2H), 7.90-7.84(m, 4H), 7.57-7.50(m, 4H), 7.47-7.43(m, 2H), 7.39(d, *J*=5.9 Hz, 2H), 7.34(t, *J*=7.4Hz, 1H), 7.27(t, *J*=7.4Hz,
1H),6.91(d,J=9.1Hz,2H),5.04(d,J=10.6 Hz, 1H), 4.97(d, *J*=7.7Hz, 1H), 4.48(d, *J*=10.7 Hz,1H), 3.89-3.82(m, 2H), 3.73(dd, *J*=3.3, 9.6 Hz, 1H).

In this example, the stability of galactopyranosides (Compounds 5 to 10) in which pyrazolone derivatives were introduced, was examined in phosphate buffer solution. (Examination of the stability of galactopyranosides in which pyrazolone derivatives were introduced)

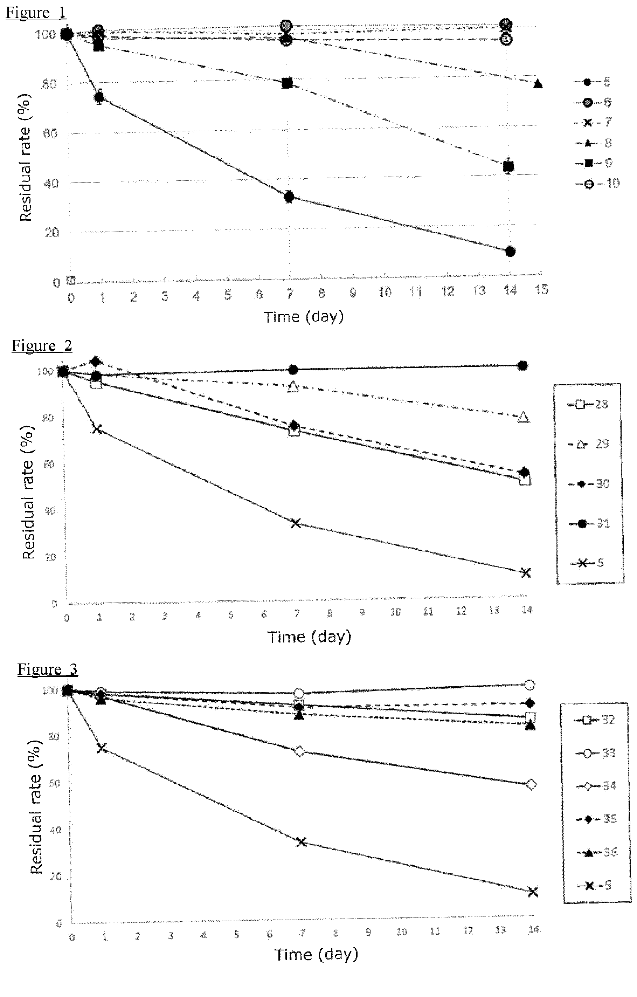

Galactopyranoside (compound 5 to 10) in which pyrazolone derivative was introduced, was dissolved in DMSO to be a solution of 5 mM. 0.1 M pH 7.4 phosphate buffer (125 µL), water (62.5 µL) and DMSO (37.5 µL) were added to 5 mM compounds 5 to 10 (25 µL), and the mixture was heated to 37°C. A portion of the reaction solution after 1 to 35 days was measured by reversed-phase HPLC, and the percentage of compounds 5 to 10 remaining was calculated from the peak area (Fig. 1).

As a result, pyrazolonated compounds 6 to 10 obtained from pyrazolone compounds in which R1 is electron-withdrawing group and formyl group-containing compounds showed improved stability compared to the control compound 5 (R¹=Me).

[HPLC analysis conditions using reversed-phase column].
HPLC: GL-7400 (GL Sciences Inc.)
Column: Mightysil RP-18 GP Aqua, 5 µm, Φ2 × 250 mm (Kanto Chemical Co. Inc.)
Column temperature: 30°C
Mobile phase A: H₂O solution containing 0.1% TFA (v/v)
Mobile phase B: MeCN solution containing 0.1% TFA (v/v)
Gradient (mobile phase B%): Compound 5, 30% (0 min), 60% (30 min), Compound 6, 45% (0 min), 80% (30 min), Compound 7, 60% (0 min), 90% (30 min), Compound 8, 50% (0 min), 80% (30 min), Compound 9, 50% (0 min), 80% (30 min), Compound 10 30% (0 min), 80% (30 min).
Flow rate: 0.2mL/min
Detection wavelength: Compound 5, 240nm, Compound 6, 240nm, Compound 7, 240nm, Compound 8, 252nm, Compound 9, 250nm, Compound 10, 235nm.

In the present invention, the compounds listed in the below Table 1 are preferable. In addition, n=0 for the pyrazolone compounds in the below Table 1.

### <<Example 10-26>>

### (Preparation of Compound 11) (Example 10)

Ethyl (4-fluorobenzoyl) acetate (300 µL, 1.70 mmol) was dissolved in acetic acid (3.0 mL) under argon atmosphere, and phenylhydrazine (183 µL, 1.87 mmol) was added thereto. After stirring at 85°C for 17 hours, ethyl acetate was added to the reaction solution, and the organic phase was washed with water, saturated sodium bicarbonate solution, and saturated saline in this order, and dried over anhydrous magnesium sulfate. The solution was filtered and removed under reduced pressure, and the residue was purified by silica gel column chromatography (hexane:ethyl acetate = 4:1) to obtain 206 mg (95% yield) of compound 11. 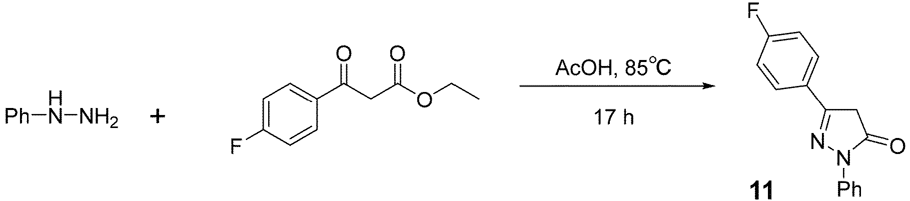 Compound 11 ¹H-NMR(600MHz, CDCl₃):δ(ppm)=7.97(dd, *J*=1.2, 9.0 Hz, 2H),7.80-7.77(m, 2H), 7.44(t, *J*=7.2 Hz, 2H), 7.23(t, *J*=7.2 Hz, 1H), 7.17-7.15(m, 2H), 3.85(s, 2H).

### (Preparation of Compound 12) (Example 11)

Ethyl (4-chlorobenzoyl) acetate (150 mg, 0.66 mmol) was dissolved in acetic acid (1.5 mL) and phenylhydrazine (72 µL, 0.73 mmol) was added thereto. After stirring at 85°C for 22 hours, ethyl acetate was added to the reaction solution, and the organic phase was washed with water, saturated sodium bicarbonate solution, and saturated saline in this order, and dried over anhydrous magnesium sulfate. The solution was filtered and removed under reduced pressure, and the residue was purified by silica gel column chromatography (hexane:ethyl acetate = 3:1) to obtain 154 mg (84% yield) of compound 12. 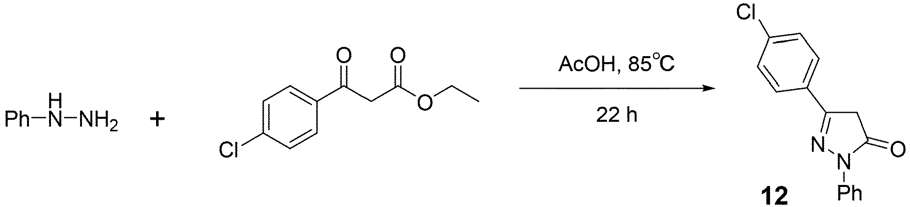 Compound 12 ¹H-NMR(600MHz, CDCl₃):δ(ppm)=7.93(d, *J*=7.8Hz, 2H), 7.68(d, *J*=8.4 Hz, 2H), 7.43-7.40(m, 4H), 7.22(t, *J*=7.2 Hz, 1H), 3.78(s, 2H).

### (Preparation of Compound 13) (Example 12)

Ethyl (4-Cyanobenzoyl) acetate (300 mg, 1.38 mmol) was dissolved in acetic acid (3.0 mL) and phenylhydrazine (150 µL, 1.52 mmol) was added thereto. After stirring at 85°C for 22 hours, ethyl acetate was added to the reaction solution, and the organic phase was washed with water, saturated sodium bicarbonate solution, and saturated brine in this order, and dried over anhydrous magnesium sulfate. The solution was filtered and removed under reduced pressure, and the residue was purified by silica gel column chromatography (hexane:ethyl acetate = 3:1) to obtain 198 mg (55% yield) of compound 13. 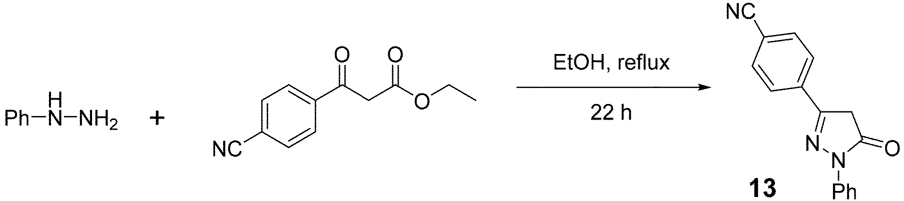 Compound 13 ¹H-NMR(600MHz, CDCl₃):δ(ppm)=7.92(d, *J*=7.8 Hz, 2H), 7.87(d, *J*=7.8 Hz, 2H), 7.74(d, *J*=8.4 Hz, 2H), 7.44(t, *J*=7.8 Hz, 2H), 7.25(t, *J*=7.8 Hz, 1H), 3.86(s, 2H).

### (Preparation of Compound 14) (Example 13)

Ethyl 3-oxo-3-(pyridin-4-yl)propanoate (215 mg, 1.11 mmol) was dissolved in tetrahydrofuran (3.7 mL) under argon atmosphere, and methylhydrazine (58.6 µL, 1.11 mmol) was added thereto. After stirring at 65°C for 5 hours, the solvent was removed under reduced pressure, and the resulting residue was purified by silica gel column chromatography (chloroform:methanol = 30:1) to obtain 181 mg (93% yield) of compound 14. 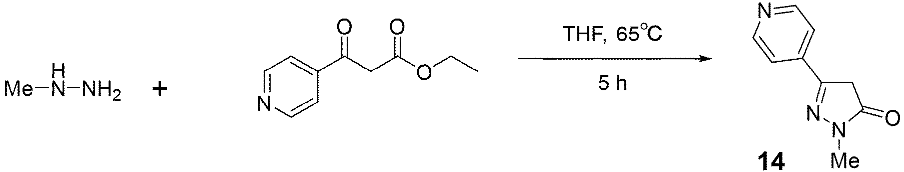 Compound 14 ¹H-NMR(600MHz, CDCl₃):δ(ppm)=8.67(d, *J*=6.2Hz, 2H), 7.51(d, *J*=6.1Hz, 2H), 3.59(s, 2H), 3.44(s, 3H).

### (Preparation of Compound 15) (Example 14)

Ethyl 3-oxo-3-(pyridin-2-yl)propanoate (155 mg, 802 µmol) was dissolved in acetic acid (1.5 mL) under argon atmosphere, and phenylhydrazine (86.8 µL, 883 µmol) was added thereto. After stirring at 85°C for 22 hours, the reaction solution was diluted with methanol. The solvent was removed under reduced pressure by toluene azeotrope, dried by vacuum pumping, and the resulting residue was purified by silica gel column chromatography (hexane:ethyl acetate = 1: 1) to obtain 152 mg (80% yield) of compound 15. 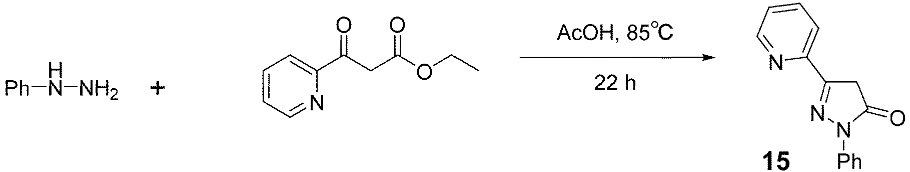 Compound 15 ¹H-NMR(600MHz, CDCl₃):δ(ppm)=8.64(d, *J*=4.8 Hz, 1H), 8.15(dd, *J*=1.2, 8.4 Hz, 1H), 7.97(d, *J*=8.4 Hz, 2H), 7.80(t, *J*=7.8 Hz, 1H), 7.45(t, *J*=7.8 Hz, 2H), 7.37-7.33(m, 1H), 7.25-7.22(m, 1H), 4.03(s, 2H).

### (Preparation of Compound 16) (Example 15)

3-(2,6-Dichloro-5-fluoropyridin-3-yl)-3-oxopropanoic acid ethyl ester (202 mg, 721 µmol) was dissolved in acetic acid (2.0 mL) and phenylhydrazine (78.0 µL, 793 µmol) was added thereto. After stirring at 85°C for 22 hours, the reaction solution was diluted with methanol. The solvent was removed under reduced pressure by toluene azeotrope, dried by vacuum pumping, and the resulting residue was purified by silica gel column chromatography (hexane:ethyl acetate = 4:1) to obtain 176 mg (75% yield) of compound 16. 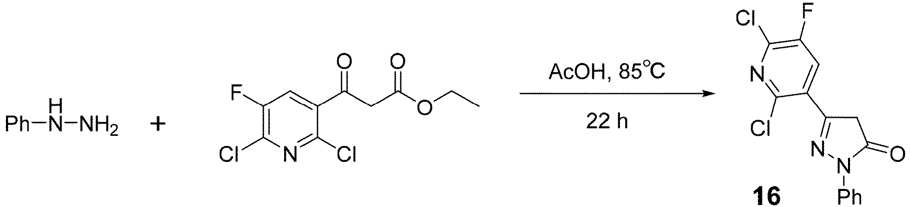 Compound 16 ¹H-NMR(600MHz, DMSO-d₆):δ(ppm)=12.13(brs, 1H), 8.41(d, *J*=8.8 Hz, 1H), 7.81(dd, *J*=1.0, 7.8 Hz, 2H), 7.49(t, *J*=7.6 Hz, 2H), 7.33(d, *J*=7.4 Hz, 1H), 6.22(s, 1H).

Compound 16 was observed as an enol form in DMSO-d₆.

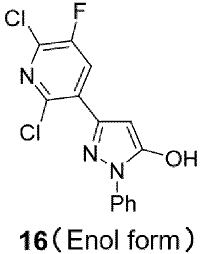

### (Preparation of Compound 17) (Example 16)

Ethyl 3-oxo-3-(pyridin-3-yl)propanoate (196 mg, 1.01 mmol) was dissolved in acetic acid (2.0 mL) and phenylhydrazine (110 µL, 1.12 mmol) was added thereto. After stirring at 85°C for 22 hours, the reaction solution was diluted with methanol. The solvent was removed under reduced pressure by toluene azeotrope, dried by vacuum pumping, and the resulting residue was purified by silica gel column chromatography (chloroform:methanol = 50:1) to obtain 169 mg (70% yield) of compound 17. 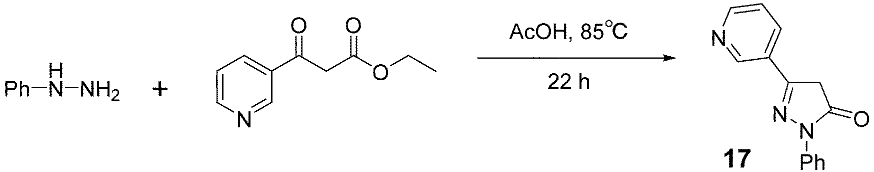 Compound 17 ¹H-NMR(600MHz, CDCl₃):δ(ppm)=8.94(d, *J*=2.0 Hz, 1H), 8.70(dd, *J*=1.1, 4.8 Hz, 1H), 8.16-8.14(m, 1H), 7.96(d, *J*=8.4Hz, 2H), 7.46-7.41(m, 3H), 7.25-7.23(m, 1H), 3.89(s, 2H).

### (Preparation of Compound 18) (Example 17)

[6-(trifluoromethyl)nicotinyl]acetic acid methyl ester(144 mg, 582 µmol) was dissolved in acetic acid (1.4 mL), and phenylhydrazine (63.0 µL, 641 µmol) was added thereto. After stirring at 85°C for 22 hours, the reaction solution was diluted with methanol. The solvent was removed under reduced pressure by toluene azeotrope, dried by vacuum pumping, and the resulting residue was purified by silica gel column chromatography (hexane:ethyl acetate = 2:1) to obtain 167 mg (94% yield) of compound 18. 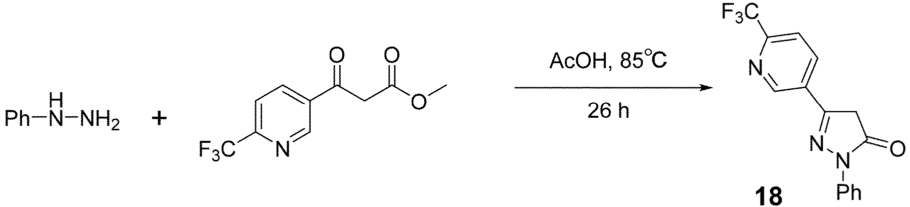 Compound 18 ¹H-NMR(600MHz, CDCl₃):δ(ppm)=9.05(brs, 1H), 8.30(d, *J*=8.2 Hz, 1H), 7.93(d, *J*=7.7 Hz, 2H), 7.79(d, *J*=8.2 Hz, 1H), 7.46(t, *J*=7.7 Hz, 2H), 7.29-7.27(m, 1H), 3.91(s, 2H).

### (Preparation of Compound 19) (Example 18)

Ethyl 3-oxo-3-(pyrazin-2-yl)propionate (417 mg, 2.15 mmol) was dissolved in acetic acid (4.2 mL) and phenylhydrazine (232 µL, 2.32 mmol) was added thereto. After stirring at 85°C for 2 hours, the reaction solution was diluted with ethyl acetate. The solvent was removed under reduced pressure by toluene azeotrope, dried by vacuum pumping, and the resulting residue was purified by silica gel column chromatography (chloroform:acetone = 5:1) to obtain 434 mg (85% yield) of compound 19. 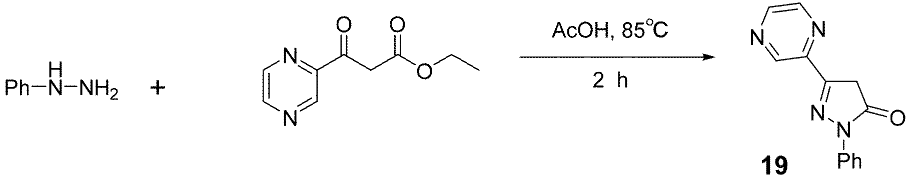 Compound 19 ¹H-NMR(600MHz, DMSO-d₆):δ(ppm)=12.13(brs, 1H), 8.65(dd, *J*=1.6, 2.4 Hz, 1H), 8.57(d, *J*=2.5Hz, 1H), 7.84(d, *J*=8.1Hz, 2H), 7.53-7.50(m, 2H), 7.35(t, *J*=7.4 Hz, 1H), 6.17(s, 1H).

Compound 19 was observed as an enol form in DMSO-d₆.

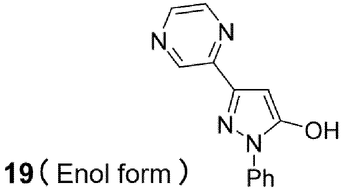

### (Preparation of Compound 20) (Example 19)

Ethyl (2,3,4,5,6-pentafluorobenzoyl)acetate (150 mg, 0.53 mmol) was dissolved in acetic acid (1.5 mL) and phenylhydrazine (57.0 µL, 0.58 mmol) was added thereto. After stirring at 80°C for 1 hour, the solvent was removed under reduced pressure by toluene azeotrope. The residue was dried by vacuum pumping, and the resulting residue was purified by silica gel column chromatography (toluene:ethyl acetate = 30: 1) to obtain 110 mg (64% yield) of compound 20. 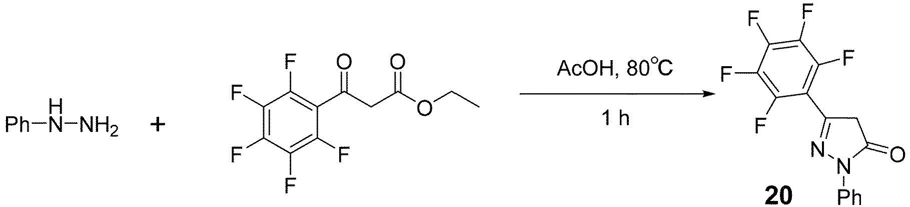 Compound 20 ¹H-NMR(600MHz, CDCl₃):δ(ppm)=7.89(d, *J*=8.2 Hz, 2H), 7.43(t, *J*=8.6 Hz, 2H), 7.26-7.23(m, 1H), 3.92(s, 2H).

### (Preparation of Compound 21) (Example 20)

Ethyl (4-chloro-2,3,5-trifluorobenzoyl)acetate (150 mg, 0.53 mmol) was dissolved in acetic acid (1.5 mL), and phenylhydrazine (58.0 µL, 0.59 mmol) was added thereto. After stirring at 80°C for 23 hours, the solvent was removed under reduced pressure by toluene azeotrope. The residue was dried by vacuum pumping, and the resulting residue was purified by silica gel column chromatography (hexane:ethyl acetate = 6:1) to obtain 136 mg (79% yield) of compound 21. 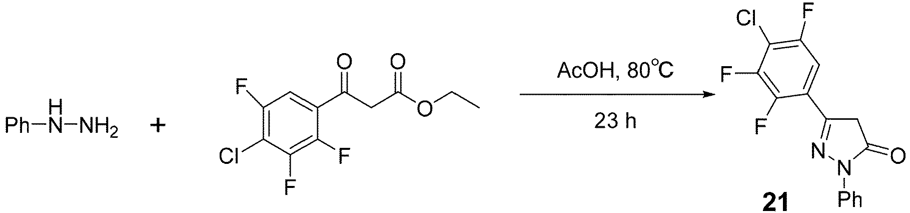 Compound 21 ¹H-NMR(600MHz, CDCl₃):δ(ppm)=7.92-7.97(m, 3H), 7.43(t, *J*=7.8 Hz, 2H), 7.26-7.23(m, 1H), 3.94(s, 2H).

### (Preparation of Compound 22) (Example 21)

Ethyl 5-[(4-methylphenyl)sulfonyl]-3-oxopentanoate (150 mg, 0.50 mmol) was dissolved in acetic acid (1.5 mL) and phenylhydrazine (54.0 µL, 0.55 mmol) was added thereto. After stirring at 80°C for 40 minutes, the solvent was removed under reduced pressure by toluene azeotrope. The residue was dried by vacuum pumping, and the resulting residue was purified by silica gel column chromatography (hexane:ethyl acetate = 3:2) to obtain 75 mg (44% yield) of compound 22. 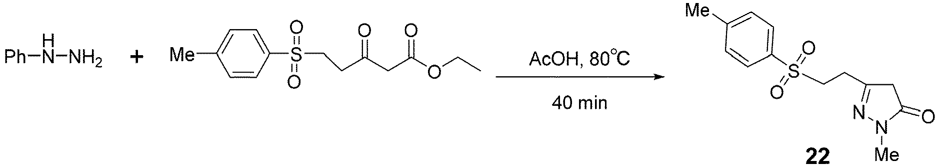 Compound 22 ¹H-NMR(600MHz, CDCl₃):δ(ppm)=7.78-7.72(m, 4H), 7.34-7.31(m, 4H), 7.14(t, *J*=7.2 Hz, 1H), 3.50(t, *J*=7.2 Hz, 2H), 3.34(s, 2H), 2.86(t, *J*=7.2 Hz, 2H), 2.38(s, 3H).

### (Preparation of Compound 23) (Example 22)

Ethyl (3-methoxybenzoyl) acetate (43.6 µL, 0.23 mmol) was dissolved in acetic acid (563 µL) under argon atmosphere, and phenylhydrazine (24.3 µL, 0.25 mmol) was added thereto. After stirring at 85°C for 24 hours, the reaction solution was diluted with ethyl acetate, and made basic by adding saturated sodium bicarbonate solution. The organic phase was washed with saturated sodium bicarbonate solution and dried over anhydrous sodium sulfate. The solution was filtered and removed under reduced pressure, and the residue was purified by silica gel column chromatography (hexane:ethyl acetate = 6:1-4:1) to obtain 46 mg (76% yield) of compound 23. 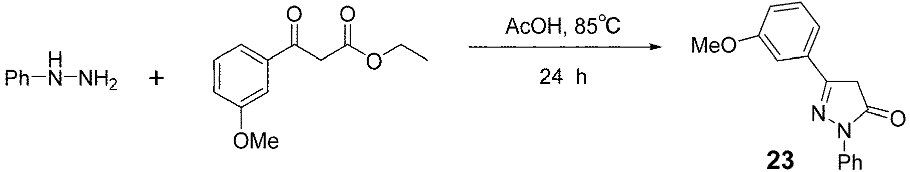 Compound 23 ¹H-NMR(600MHz, CDCl₃):δ(ppm)=7.97(d, *J*=7.6 Hz, 2H), 7.43(t, *J*=7.5Hz, 2H), 7.36-7.35(m, 2H), 7.28(d, *J*=7.6 Hz, 1H), 7.22(t, *J*=7.4 Hz, 1H), 7.00(dd, *J*=2.6, 8.0 Hz, 1H), 3.88(s, 3H), 3.38(s, 2H).

### (Preparation of Compound 24) (Example 23)

Ethyl (2-Nitrobenzoyl) acetate (600 mg, 2.53 mmol) was dissolved in acetic acid (6.0 mL) and phenylhydrazine (273 µL, 2.79 mmol) was added thereto. After stirring at 80°C for 21 hours, the solvent was removed under reduced pressure by toluene azeotrope. The residue was dried by vacuum pumping, and the resulting residue was purified by silica gel column chromatography (hexane:ethyl acetate = 4:1) to obtain 104 mg (15% yield) of compound 24. 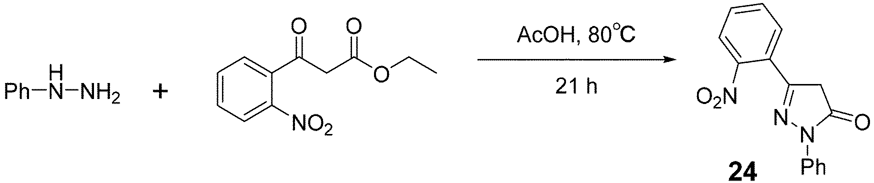 Compound 24 ¹H-NMR(600MHz, DMSO-d₆):δ(ppm)=12.04(brs, 1H), 7.85(dd, *J*=7.5 Hz, 1H), 7.7-7.69(m, 3H), 7.58(t, *J*=7.8 Hz, 1H), 7.49(t, *J*=7.8Hz, 2H), 7.31(t, *J*=7.5Hz, 1H), 5.86(s, 1H).

Compound 24 was observed as an enol form in DMSO-d₆.

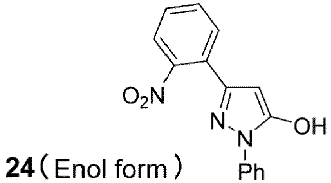

### (Preparation of Compound 25) (Example 24)

Ethyl (3-nitrobenzoyl)acetate (300 mg, 1.26 mmol) was dissolved in acetic acid (4.0 mL) and phenylhydrazine (137 µL, 1.39 mmol) was added thereto. After stirring at 80°C for 21 hours, the solvent was removed under reduced pressure by toluene azeotrope. The residue was dried by vacuum pumping, and the resulting residue wapurified by silica gel column chromatography (hexane:ethyl acetate = 3:1) to obtain 242 mg (68% yield) of compound 25. 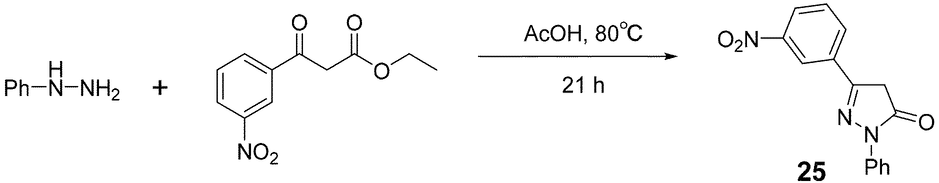 Compound 25 ¹H-NMR(600MHz, CDCl₃):δ(ppm)=7.94(d, *J*=7.8 Hz, 2H), 7.69(t, *J*=8.4 Hz, 2H), 7.44-7.41(m, 4H), 7.22(t, *J*=7.2 Hz, 1H), 3.79(s, 2H).

### (Preparation of Compound 26) (Example 25)

Ethyl hydrazinoacetate hydrochloride (1.04 g, 6.74 mmol) and sodium acetate (615 mg, 7.50 mmol) were dissolved in anhydrous ethanol (10 mL) under argon atmosphere. After stirring at room temperature for 5 min, ethyl 4,4,4-trifluoroacetoacetate (1.00 mL, 6.80 mmol ) was added and the mixture was heated to reflux for 24 hours.

The insoluble matter was filtered off, the filtrate was concentrated under reduced pressure, and the resulting residue was purified by silica gel column chromatography (chloroform:methanol:acetic acid = 190:10:2) to obtain the crude product of ethyl ester A. The resulting crude product was heated and dissolved in ethyl acetate and precipitated with hexane to obtain 513 mg (32% yield) of ethyl ester A. After dissolving 242 mg (1.01 mmol) of the ethyl ester A in a mixture solvent of tetrahydrofuran (6.8 mL) and methanol (10.2 mL), sodium hydroxide solution (603 mM, 3.4 mL) was added thereto, and the mixture was stirred at room temperature for 19 hours. 4 M hydrochloric acid was added to the reaction solution under ice-cold conditions to bring the reaction solution to pH 6.0, and the solvent was removed under reduced pressure. The resulting residue was purified by silica gel column chromatography (chloroform:methanol = 2:1-1:4) to obtain the crude product of carboxylic acid form A (268 mg).

The resulting crude product of carboxylic acid form A was dissolved in DMF (1 mL) under argon atmosphere, and then 1-hydroxy-7-azabenzotriazole (HOAt: 209 mg, 1.53 mmol), 11-azido-3,6,9-trioxaundecan-1-amine (250 µL, 1.26 mmol), N,N-diisopropylethylamine (260 µL, 1.53 mmol), 1-(3-dimethylaminopropyl)-3-ethylcarbodiimide hydrochloride (EDC-HCl: 300 mg, 1.57 mmol) in DMF (1.5 mL) was added thereto, and the mixture was stirred at room temperature for 23 hours.

The solvent was removed under reduced pressure, and the resulting residue was purified by silica gel column chromatography (chloroform:methanol = 40:1-35:1) to obtain 283 mg (yield 67%, 2 steps) of compound 26.

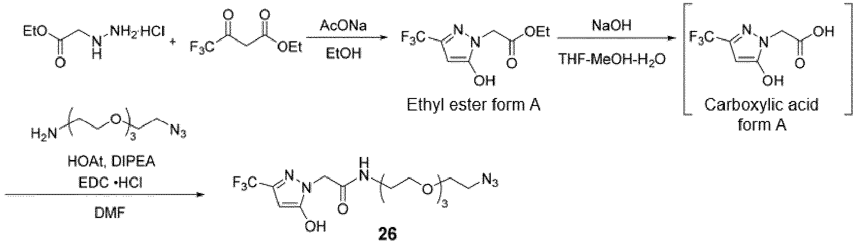

Compound 26 ¹H-NMR(600MHz, DMSO-D6):δ(ppm)=11.82(brs, 1H), 8.16(t, *J*=5.5 Hz, 1H), 5.71(s, 1H), 4.59(s, 2H), 3.64-3.59(m, 2H), 3.58-3.51(m, 8H), 3.47-3.43(m, 2H), 3.40-3.38(m, 2H), 3.28-3.22(m, 2H).

### (Preparation of Compound 27) (Example 26)

Ethyl hydrazinoacetate hydrochloride (1.04 g, 6.72 mmol) and sodium acetate (607 mg, 7.40 mmol) were dissolved in anhydrous ethanol (20 mL) under argon atmosphere. After stirring at room temperature for 5 min, ethyl 3-oxo-3-(4-pyridyl)propionate (1.43 g, 7.41 mmol) was added thereto, and the mixture was heated to reflux for 22 hours. The insoluble matter was filtered off, the filtrate was concentrated under reduced pressure, and the resulting residue was purified by silica gel column chromatography (chloroform:methanol = 40:1-25:1) to obtain 569 mg (34% yield) of ethyl ester C. After dissolving 301 mg (1.22 mmol) of ethyl ester C in a mixture solvent of tetrahydrofuran (8.2 mL) and methanol (12.3 mL), sodium hydroxide solution (603 mM, 4.1 mL) was added thereto, and the mixture was stirred at room temperature for 19 hours. 4 M hydrochloric acid was added to the reaction solution under ice-cold conditions to bring the reaction solution to pH 6.0, and the solvent was removed under reduced pressure. The resulting residue was purified by silica gel column chromatography (chloroform:methanol = 3:1-1;1) to obtain the crude product of carboxylic acid form C (316 mg). The resulting crude product was desalted by Diaion HP-20 to obtain 190 mg (71% yield) of carboxylic acid product C. 98 mg (466 µmol) of the resulting carboxylic acid form C was dissolved in DMF (1.0 mL) under argon atmosphere, and 1-hydroxy-7-azabenzotriazole (HOAt: 100 mg, 735 µmol), 11-azido-3,6,9-trioxaundecan-1-amine (110 µL, 554µmol), N,N-diisopropylethylamine (115 µL, 676 µmol), 1-(3-dimethylaminopropyl)-3-ethylcarbodiimide hydrochloride (EDC-HCl: 212 mg, 678 µmol) in DMF solution (1.5 mL) was added thereto, and the mixture was stirred at room temperature for 19 hours. The solvent was removed under reduced pressure, and the resulting residue was purified by silica gel column chromatography (chloroform:methanol = 40:1-10:1) to obtain the crude product of compound 27. The crude product was purified again by silica gel column chromatography (chloroform:methanol:acetic acid = 250:10:0.26-200:10:0.21) to obtain 43 mg (23% yield) of compound 27.

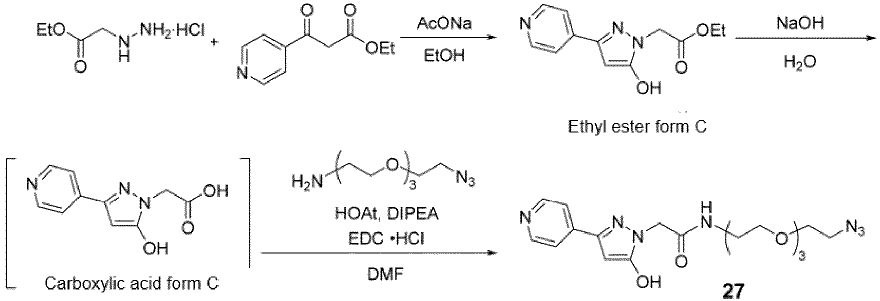

Compound 27 ¹H-NMR(600MHz,CD₃OD):δ(ppm)=8.52(brs, 2H), 7.80-7.76(m, 2H), 4.72(s, 2H), 3.65-3.59(m, 12H), 3.59-3.55(m, 2H), 3.42(t, *J*=5.4 Hz, 2H), 3.37-3.34(m, 2H).

In the following Examples, pyrazolone compounds were introduced into galactopyranosides and pyrazolone-galactose complexes were synthesized.

### <<Examples 27 to 41>> (Common synthesis Examples)

The syntheses of compounds 28 to 42, pyrazolone-galactose complexes, were carried out according to the following procedure.

An aqueous solution of 4-nitrophenyl β-D-galactopyranoside (50 mM, 720 µL), phosphate buffer (1.0 M, pH 7.0, 1.8 mL), water (32.5 mL), galactose oxidase solution (Worthington Biochemical Corporation, 90 units/mL, 623µL), horseradish peroxidase (Oriental Yeast Co., Ltd, 3 units/mL, 396µL), DMF solution of pyrazolone compound (25 mM, 5.76 mL) were added in a 50 mL polypropylene centrifuge tube, and the mixture is shaken at 30°C, a shaking rate of 165 times/min for 24 hours, in a shaking constant temperature bath. The reaction solution is transferred to a 1 L of eggplant flask, methanol is added thereto, and the solvent is concentrated under reduced pressure. After drying by vacuum pumping, the resulting residue is dissolved in methanol and the insoluble matter is filtered off. The resulting filtrate is concentrated under reduced pressure, and the resulting residue is purified to obtain pyrazolone-galactose complex.

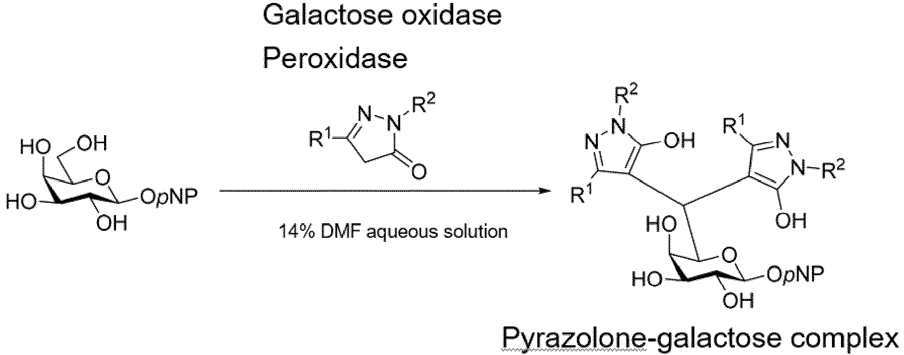

### (Preparation of Compound 28) (Example 27)

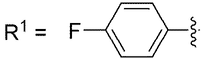

R² = Ph
Raw material pyrazolone compound: Compound 11
Yield: 22 mg (79% yield)
Purification conditions: Silica gel column chromatography (chloroform:methanol=20:1) Compound 28 ¹H-NMR(600MHz, CD₃OD):δ(ppm)=7.85(d, *J*=7.8 Hz, 2H), 7.83(d, *J*=7.8 Hz, 2H), 7.75(d, *J*=7.8 Hz, 2H), 7.55(t, *J*=7.8 Hz, 2H), 7.49-7.46(m, 4H), 7.38(t, *J*=7.8 Hz, 1H), 7.32(t, *J*=7.8 Hz, 1H), 7.22(dd, *J*=5.4, 8.6 Hz, 2H), 6.95(d, *J*=9.3 Hz, 2H), 6.91(t, *J*=8.8 Hz, 2H), 6.88(t, *J*=8.8 Hz, 1H), 5.01(d, *J*=7.8 Hz,1H),4.98(d, *J*=10.9 Hz, 1H), 4.47(d, *J*=10.8 Hz, 1H), 3.87-3.84(m, 2H), 3.70(dd, *J*=3.3, 9.6 Hz, 1H).

### (Preparation of Compound 29) (Example 28)

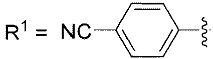

R² = Ph
Raw material pyrazolone compound: Compound 13
Yield: 20 mg (70% yield)
Purification conditions: Silica gel column chromatography (chloroform:methanol=20:1-7:1)
Compound 29 ¹H-NMR(CD₃OD):δ(ppm)=7.91-7.82(m, 4H), 7.78(d, *J*=7.5Hz, 2H), 7.62(d, *J*=8.1 Hz, 2H),7.58-7.53(m, 4H), 7.50-7.30(m, 4H), 6.99-6.92(m, 1H), 5.04(d, *J*=7.8 Hz, 1H),5.03(d, *J*=10.5 Hz, 1H), 4.45(d, *J*=10.8 Hz, 1H),3.90-3.85(m, 2H), 3.72(dd, *J*=9.6, 3.3Hz, 1H), 3.64-3.59(m, 1H).

### (Preparation of Compound 30) (Example 29)

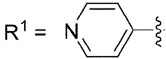

R² = Me
Raw material pyrazolone compound: Compound 14
Yield: 21 mg (91% yield)
Purification conditions: Silica gel column chromatography (chloroform:methanol==5:1-1: 1)
Compound 30 ¹H-NMR(CD₃OD):δ(ppm)=8.31(d, *J*=5.5 Hz, 2H), 8.19(d, *J*=5.6Hz, 2H), 8.03-8.01(m, 2H),7 .39(d, *J*=6.2 Hz, 2H), 7.29(d, *J*=6.1 Hz, 2H), 6.89(d, *J*=9.3 Hz, 2H), 4.95(d, *J*=7.7 Hz, 1H), 4.39(d, *J*=10.6 Hz, 1H), 3.82(dd, *J*=7.8, 9.6 Hz, 1H), 3.74(s, 3H), 3.71(d, *J*=2.9 Hz, 1H),3.66(dd, *J*=3.3, 9.6 Hz, 1H), 3.63(s, 3H).

### (Preparation of Compound 31) (Example 30)

R¹ = CF₃

R² = Me

Yield: 11 mg (52% yield)
Raw material pyrazolone compound: 1-methyl-3-trifluoromethyl-5-pyrazolone
Purification conditions: Silica gel column chromatography (chloroform:methanol = 5 :1-1:1) followed by silica gel column chromatography again (chloroform:methanol = 5:1-2:1). The crude product was subjected to preparative thin-layer chromatography (20 x 20 cm, Developing solvent: chloroform:methanol=2:1).
Compound 31 ¹H-NMR(CD₃OD):δ(ppm)=8.02(d, *J*=9.2 Hz,2 H), 6.84(d, *J*=9.2 Hz, 2H), 4.82(d, *J*=7.8 Hz, 1H), 4.68(d, *J*=10.7 Hz, 1H), 4.52(d, *J*=10.7 Hz, 1H), 3.82(dd, *J*=7.8, 9.6 Hz, 1H), 3.67(s, 3H), 3.63(d, *J*=2.9 Hz, 1H), 3.57(dd, *J*=3.2, 9.5 Hz, 1H), 3.54(s, 3H), 3.45(s, 1H), 3.35(s, 1H).

### (Preparation of Compound 32) (Example 31)

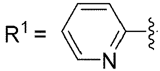

R² = Ph
Raw material pyrazolone compound: Compound 15
Yield: 23 mg (85% yield)
Purification conditions: Silica gel column chromatography (chloroform:methanol=20:1-8:1)
Compound 32 MALDI-TOF MS. :Calcd for C₄₀H₃₄N₇O₉ m/z[M+H]⁺:756.241, Found 756.165.

### (Preparation of Compound 33) (Example 32)

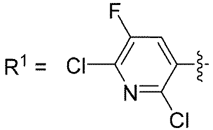

R² = Ph
Raw material pyrazolone compound: Compound 16
Yield: 30 mg (89% yield)
Purification conditions: Silica gel column chromatography (chloroform:methanol =20:1-8:1) followed by silica gel column chromatography again (chloroform:methanol =20:1-5:1).
Compound 33 ¹H-NMR(600 MHz, CD₃OD):δ(ppm)=8.16(d, *J*=8.1 Hz, 1H), 7.88(d, *J*=7.8 Hz, 2H), 7.84(d, *J*=8.0 Hz,2H), 7.77(d, *J*=8.0 Hz, 2H), 7.56-7.29(m, 9H), 6.95(d, *J*=9.2 Hz, 2H), 5.03(d, *J*=7.8 Hz, 1H), 3.86(dd, *J*=7.8, 9.5 Hz, 1H), 3.82(d, *J*=3.2 Hz, 1H), 3.71(dd, *J*=3.4, 9.6 Hz, 1H), 3.64-3.59(m, 3H).

### (Preparation of Compound 34) (Example 33)

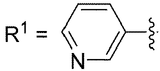

R² = Ph
Raw material pyrazolone compound: Compound 17
Yield: 27 mg (99% yield)
Purification conditions: Silica gel column chromatography (chloroform: methanol: water = 80:25:5)
Compound 34 ¹H-NMR(600MHz, CD₃OD):δ(ppm)=7.89-7.83(m, 5H), 7.79(d, *J*=7.2 Hz, 2H), 7.59(d, *J*=7.8 Hz, 1H), 7.52(t, *J*=7.8 Hz, 2H), 7.45(t, *J*=7.8 Hz, 2H), 7.35(t, *J*=7.2 Hz, 1H), 7.28(t, *J*=7.2 Hz, 1H), 7.18(brs, 1H), 7.08(brs, 1H), 6.92(d, *J* =9.0 Hz, 2H), 5.00-4.98(m, 2H), 4.28(d, *J*=10.8 Hz, 1H), 3.89-3.83(m, 2H), 3.70(dd, *J*=3.6, 9.6 Hz, 1H), 3.60(s, 1H).

### (Preparation of Compound 35) (Example 34)

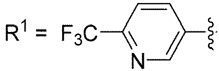

R² = Ph
Raw material pyrazolone compound: Compound 18
Yield: 16 mg (51% yield)
Purification conditions: Silica gel column chromatography (chloroform:methanol:water =85:20:1)
Compound 35 ¹H-NMR(600MHz, CD₃OD):δ(ppm)=8.81(d, *J*=1.2 Hz, 1H), 8.62(d, *J*=1.2 Hz, 1H), 7.98(dd, *J*=2.4, 7.8 Hz, 1H), 7.91-7.81(m, 7H), 7.55-7.52(m, 3H), 7.47-7.44(m, 2H), 7.37-7.34(m, 2H), 7.31-7.28(m, 1H), 5.01-4.99(m, 2H), 4.25(d, *J*=10.8 Hz, 1H), 3.58-3.82(m, 2H), 3.71(dd, *J*=3.6, 9.6 Hz, 1H).

### (Preparation of Compound 36) (Example 35)

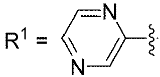

R² = Ph
Raw material pyrazolone compound: Compound 19
Yield: 22 mg (81% yield)
Purification conditions: Silica gel column chromatography (chloroform:methanol:water =80:25:5)
Compound 36 ¹H-NMR(600MHz, CD₃OD):δ(ppm)=9.16(d, *J*=1.8 Hz, 1H), 8.71(d, *J*=2.4 Hz, 1H), 8.64(s, 1H), 8.61(s, 1H), 8.18(d, *J*=2.4 Hz, 1H), 7.99(d, *J*=7.8 Hz, 2H), 7.88(d, 2H), 7.80-7.76(m, *J*=7.8 Hz, 2H), 7.56(brs, 1H), 7.51(t, *J*=8.4 Hz, 2H), 7.46(t, *J*=8.4 Hz, 2H), 7.33(t, *J*=7.8 Hz, 1H), 7.28(t, *J*=7.8 Hz, 1H), 6.93-6.90(m, 2H), 5.42(d, *J*=12 Hz, 1H), 4.95-4.88(m, 3H), 4.09(d, *J*=3.6 Hz, 1H), 3.81(dd, *J*=7.8, 9.6 Hz, 1H), 3.71(dd, *J*=3.6, 9.6 Hz, 1H), 3.59(brs, 1H).

### (Preparation of Compound 37) (Example 36)

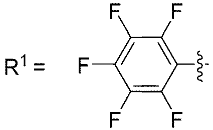

R² = Ph
Raw material pyrazolone compound: Compound 20
Yield: 31 mg (92% yield)
Purification conditions: Silica gel column chromatography (chloroform:methanol:water =85:20:1)
Compound 37 ¹H-NMR(600MHz, CD₃OD):δ(ppm)=7.83-7.77(m, 3H), 7.73(d, *J*=7.8 Hz, 2H), 7.60(d, *J*=7.8 Hz, 1H), 7.51(t, *J*=7.8 Hz, 2H), 7.46(t, *J*=7.8 Hz, 2H), 7.43-7.35(m, 2H)7.32-7.28(m, 1H), 6.92(d, *J*=9.6 Hz, 2H), 4.89(d, *J*=7.8 Hz, 1H), 4.67(d, *J*=10.8 Hz, 1H), 3.72(d, *J*=7.8, 9.6 Hz, 1H), 3.64-3.56(m, 5H), 3.32(s, 1H).

### (Preparation of Compound 38) (Example 37)

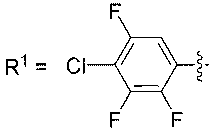

R² = Ph
Raw material pyrazolone compound: Compound 21
Yield: 26mg (78% yield)
Purification conditions: Silica gel column chromatography (chloroform:methanol:water =85:15:1)
Compound 38 ¹H-NMR(600MHz, CD₃OD):δ(ppm)=7.89-7.86(m, 2H), 7.79(d, *J*=7.8 Hz, 2H), 7.74-7.70(m, 3H), 7.55(dd, *J*=1.2, 7.2 Hz, 2H), 7.74(dd, *J*=1.2, 7.2 Hz, 2H), 7.40(t, *J*=7.8 Hz, 1H), 7.34(t, *J*=7.8 Hz, 1H), 7.05(brs, 1H), 6.96-6.93(m, 2H), 5.04(d, *J*=10.8 Hz, 1H), 3.99(d, *J*=7.8 Hz, 1H), 3.85(dd, *J*=1.8, 9.6 Hz, 1H), 3.74(d, *J*=3.0 Hz, 1H), 3.67(dd, *J*=3.0, 9.6 Hz, 1H), 3.59(s, 1H).

### (Preparation of Compound 39) (Example 38)

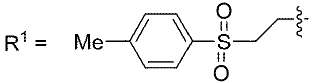

R² = Ph
Raw material pyrazolone compound: Compound 22
Yield: 31mg (89% yield)
Purification conditions: Silica gel column chromatography (chloroform:methanol:water =85:20:1)
Compound 39 ¹H-NMR(600MHz, CD₃OD):δ(ppm)=7.77(d, *J*=9.3 Hz, 2H), 7.71(t, *J*=8.3 Hz, 4H), 7.57(d, *J*=13.7 Hz, 2H), 7.54-7.50(m, 4H), 7.41-7.39(m, 4H), 7.34(t, *J*=7.4 Hz, 1H), 7.25(t, *J*=7.4 Hz, 1H), 7.17(d, *J*=8.1 Hz, 2H), 6.73(d, *J*=9.2 Hz, 2H), 4.92(d, *J*=7.7 Hz, 1H), 4.51(d, *J*=10.6 Hz, 1H), 3.87(d, *J*=10.8 Hz, 1H), 3.82(dd, *J*=7.8, 9.4 Hz, 1H), 3.63-3.61(m, 1H), 3.58-3.52(m, 3H), 3.39-3.34(m, 1H), 3.02-2.91(m, 3H), 2.83-2.78(m, 1H), 2.74-2.69(m, 1H), 2.43(s, 3H), 2.23(s, 3H).

### (Preparation of Compound 40) (Example 39)

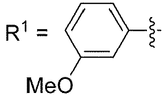

R² = Ph
Raw material pyrazolone compound: Compound 23
Yield: 20mg (70% yield)
Purification conditions: Silica gel column chromatography (chloroform:methanol=20:1) followed by silica gel column chromatography again (chloroform:methanol=50:1-30:1). The crude product was subjected to preparative thin-layer chromatography (20 x 20 cm, Developing solvent: chloroform:methanol = 7:1) followed by silica gel column chromatography (chloroform:methanol = 30:1-20:1).
Compound 40 ¹H-NMR(600MHz, CD₃OD):δ(ppm)=7.91-7.88(m, 2H), 7.85-7.84(m, 2H), 7.77-7.75(m, 2H), 7.57-7.54(m, 2H), 7.50-7.47(m, 2H), 7.40-7.38(m, 1H), 7.55-7.32(m, 1H), 7.30(brs, 1H), 7.02(t, *J*=7.7 Hz, 1H), 6.98-6.96(m, 2H), 6.91-6.90(m, 1H), 6.86-6.81(m, 3H), 6.68(d, *J*=7.5 Hz, 1H), 5.02(d, *J*=7.8 Hz, 1H), 4.98(d, *J*=10.9 Hz, 1H), 4.72(d, *J*=10.9 Hz, 1H), 3.87-3.84(m, 2H), 3.73(s, 3H), 3.70(dd, *J*=3.4, 9.6 Hz, 1H), 3.65(s, 3H).

### (Preparation of Compound 41) (Example 40)

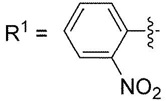

R² = Ph
Raw material pyrazolone compound: Compound 24
Yield: 15mg (48% yield)
Purification conditions: Silica gel column chromatography (chloroform:methanol=10:1-5:1) followed by silica gel column chromatography again (chloroform:methanol=10:1). The crude product was subjected to silica gel column chromatography (chloroform:methanol =50:1-20:1).
Compound 41 ¹H-NMR(600MHz, CD₃OD):δ(ppm)=7.99(d, *J*=8.1 Hz, 2H), 7.93(d, *J*=7.9 Hz, 2H), 7.82(d, *J*=9.3 Hz, 2H), 7.69(d, *J*=7.5 Hz, 2H), 7.54(t, *J*=8.2 Hz, 3H), 7.47(t, *J*=8.1 Hz, 3H), 7.45-7.33(m, 5H), 7.05(brs, 1H), 6.99(d, *J*=7.7 Hz, 2H), 5.04(d, *J*=7.8 Hz, 1H), 4.95(d, *J*=10.9 Hz, 1H), 4.16(d, *J*=10.9 Hz, 1H), 3.91(d, *J*=3.0 Hz, 1H), 3.83(dd, *J*=7.8, 9.6 Hz, 1H), 3.70(dd, *J*=3.4, 9.6 Hz, 1H).

### (Preparation of Compound 42) (Example 41)

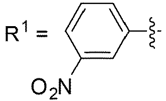

R² = Ph
Raw material pyrazolone compound: Compound 25
Yield: 21mg (68% yield)
Purification conditions: Silica gel column chromatography (chloroform:methanol=20:1-10:1)
Compound 42 MALDI-TOF MS. :calcd. for C₄₂H₃₃N₇NaO₁₃, 866.203, m/z[M+Na]⁺ found 866.206.

### <<Example 42-44>> (Other preparation methods)

### (Synthesis of Compound 43) (Example 42)

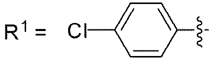

R² = Ph

Compound 12 (32.4 mg, 120 µmol) and DMF (1.0 mL) were added to 4-nitrophenyl β-D-galacto-hexodialdo-1,5-pyranoside (9.5 mg, 29.9 µmol) was added in this order. After stirring at room temperature for 1 hour, the reaction solution was concentrated under reduced pressure. The residue was purified by silica gel column chromatography (chloroform:methanol = 15:15) to obtain 23 mg (92% yield) of compound 43. Compound 43 ¹H-NMR(600MHz, CD₃OD):δ(ppm)=7.86(d, J*=*9.3 Hz, 2H), 7.83(d, *J*=7.6 Hz, 2H), 7.75(d, *J*=7.6 Hz, 2H), 7.55(t, *J*=8.0 Hz, 2H), 7.56-7.43(m, 4H), 7.38(t, *J*=7.5 Hz, 1H), 7.32(t, *J*=6.5 Hz, 1H), 7.19-7.14(m, 6H), 6.96-6.93(m, 2H), 5.00(d, *J*=7.7 Hz, 1H), 4.97(d, *J*=10.8 Hz, 1H), 4.45(d, *J*=10.8 Hz, 1H), 3.87-3.82(m, 2H), 3.70(dd, *J*=3.4, 9.7 Hz, 1H), 3.62-3.59(m, 1H).

### (Preparation of Compound 44) (Example 43)

R¹ = CF₃

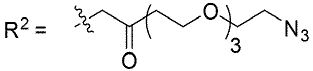

4-Nitrophenyl β-D-galacto-hexodialdo-1,5-pyranoside (23.0 mg, 72.5 µmol) was dissolved in water (7.1 mL), and compound 26 (117.1 mg, 285 µmol) in DMF (4.6 mL) was added thereto. After stirring at 37°C for 25 hours, the reaction solution was concentrated under reduced pressure. The residue was purified by silica gel column chromatography (chloroform:methanol = 10:1-3:1) to obtain 21 mg (27% yield) of compound 44.
Compound 44 MALDI-TOF MS. :calcd. for C₄₀H₅₃F₆N₁₃NaO₁₇, 1124.348, m/z[M+Na]⁺ found 1124.272.

### (Preparation of Compound 45) (Example 44)

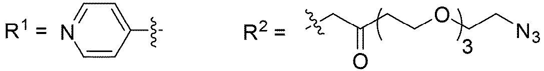

Compound 27 (25.1 mg, 59.8 µmol) and DMF (1.0 mL) were added to 4-nitrophenyl β-D-galacto-hexodialdo-1,5-pyranoside (4.8 mg, 15.0 µmol) in this order. After stirring at room temperature for 24 hours, the reaction solution was concentrated under reduced pressure. The residue was purified by silica gel column chromatography (chloroform:methanol = 10:1-3:2) to obtain 17 mg (99% yield) of compound 45. Compound 45 MALDI-TOF MS. :calcd. for C₄₈H₆₁N₁₅NaO₁₇, 1142.426, m/z[M+Na]⁺ found 1142.337.

### << Stability test >>

The stability of galactopyranosides (Compounds 28 to 45) in which pyrazolone derivatives were introduced, obtained in Examples 27 to 44 were examined in phosphate buffer.

### (Examination of the stability of galactopyranosides in which pyrazolone derivatives were introduced)

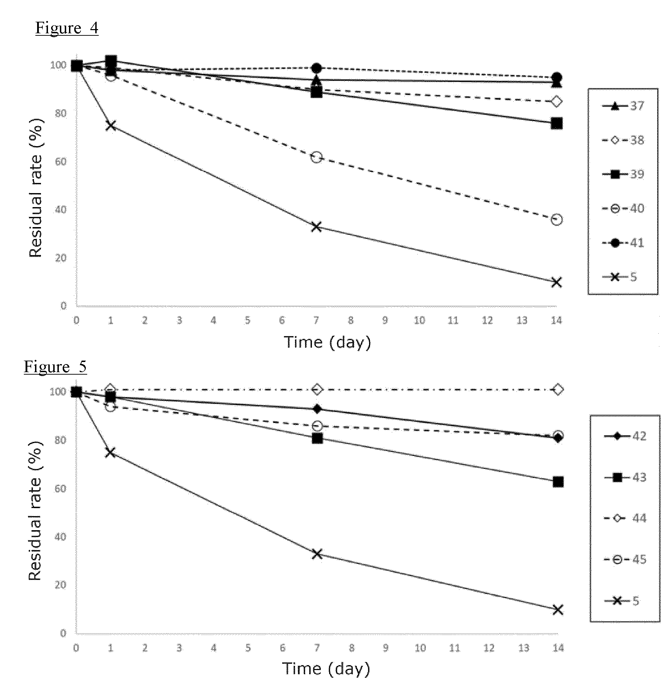

Galactopyranosides (compounds 28 to 45) in which pyrazolone was introduced, were dissolved in DMSO to be a 5 mM solution. To the 5 mM solution (75 µL), 0.1 M phosphate buffer (pH 7.4, 375 µL), water (187.5 µL) and DMSO (112.5 µL) were added, and the mixture was heated to 37°C. After 1 to 14 days, a portion of the reaction solution was measured by reversed-phase HPLC, and the residual rate of compounds 28 to 45 was calculated from the peak area (Fig. 2 to Fig. 5). As a result, it is clear that pyrazolonated compounds 28-45 obtained from the pyrazolone compound in which R1 is an electron-withdrawing group, and the formyl group-containing compound exhibit improved stability compared with the control compound, compound 5 (R¹=Me).

[HPLC analysis conditions using reversed-phase column]
(Compounds 28 to 45)
HPLC: GL-7700 (GL Sciences Inc.)
Column: Inert sustain AQ-18, 5 µm, Φ4.6 × 250 mm (GL Sciences Inc.)
Column temperature: 30°C
Mobile phase A: H₂O solution containing 0.1% TFA (v/v)
Mobile phase B: MeCN solution containing 0.1% TFA (v/v)
Gradient (mobile phase B%): Compound 28, Compound 29, Compound 35, Compound 39, Compound 40, Compound 42, Compound 43, 50%(0 min), 80%(30 min), Compound 30, 10%(0 min), 40%(30 min), Compound 31, Compound 45, 20%(0 min), 50%(30 min) Compound 32, Compound 34, Compound 44, 30%(0 min), 60%(30 min), Compound 33, 60%(0 min), 90%(30 min), Compound 36, Compound 41, 40%(0 min), 70%(30 min), Compound 37, Compound 38, 65%(0 min), 95%(30 min).
Flow rate: 1.0mL/min
Detection wavelength: Compound 28, 262nm, Compound 29, 277nm, Compound 30, 270nm, Compound 31, 226nm. Compound 32, 288nm, Compound 33, 283nm, Compound 34, 274nm, Compound 35, 275nm, Compound 36, 290nm, Compound 37, 245nm, Compound 38, 252nm, Compound 39, 250nm, Compound 40, 262nm, Compound 41, 250nm, Compound 42, 260nm, Compound 43, 265nm, Compound 44, 303nm, Compound 45, 295nm.

**[Table 1-1]**

| Compound No. | R¹ | R² |
|---|---|---|
| 1 | 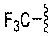 | Hydro carbon group having 1 to 1000 carbon atom which may contain a hetero atom |
| 2 | 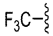 | Hydro carbon group having 1 to 200 carbon atom which may contain a hetero atom |
| 3 | 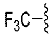 | Hydro carbon group having 1 to 100 carbon atom which may contain a hetero atom |
| 4 | 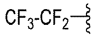 | Hydro carbon group having 1 to 1000 carbon atom which may contain a hetero atom |
| 5 | 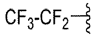 | Hydro carbon group having 1 to 200 carbon atom which may contain a hetero atom |
| 6 | 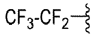 | Hydro carbon group having 1 to 100 carbon atom which may contain a hetero atom |
| 7 | 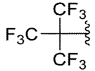 | Hydro carbon group having 1 to 1000 carbon atom which may contain a hetero atom |
| 8 | 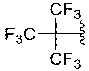 | Hydro carbon group having 1 to 200 carbon atom which may contain a hetero atom |
| 9 | 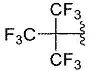 | Hydro carbon group having 1 to 100 carbon atom which may contain a hetero atom |
| 10 | 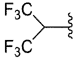 | Hydro carbon group having 1 to 1000 carbon atom which may contain a hetero atom |
| 11 | 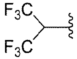 | Hydro carbon group having 1 to 200 carbon atom which may contain a hetero atom |
| 12 | 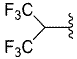 | Hydro carbon group having 1 to 100 carbon atom which may contain a hetero atom |
| 13 | 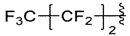 | Hydro carbon group having 1 to 1000 carbon atom which may contain a hetero atom |
| 14 | 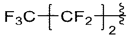 | Hydro carbon group having 1 to 200 carbon atom which may contain a hetero atom |
| 15 | 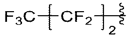 | Hydro carbon group having 1 to 100 carbon atom which may contain a hetero atom |
| 16 | 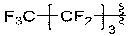 | Hydro carbon group having 1 to 1000 carbon atom which may contain a hetero atom |
| 17 | 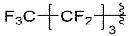 | Hydro carbon group having 1 to 200 carbon atom which may contain a hetero atom |
| 18 | 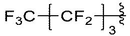 | Hydro carbon group having 1 to 100 carbon atom which may contain a hetero atom |
| 19 | 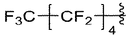 | Hydro carbon group having 1 to 1000 carbon atom which may contain a hetero atom |
| 20 | 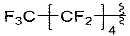 | Hydro carbon group having 1 to 200 carbon atom which may contain a hetero atom |
| 21 | 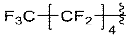 | Hydro carbon group having 1 to 100 carbon atom which may contain a hetero atom |
| 22 | 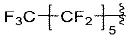 | Hydro carbon group having 1 to 1000 carbon atom which may contain a hetero atom |

**[Table 1-2]**

| Compound No. | R¹ | R² |
|---|---|---|
| 23 | 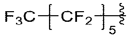 | Hydro carbon group having 1 to 200 carbon atom which may contain a hetero atom |
| 24 | 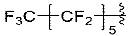 | Hydro carbon group having 1 to 100 carbon atom which may contain a hetero atom |
| 25 | 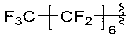 | Hydro carbon group having 1 to 1000 carbon atom which may contain a hetero atom |
| 26 | 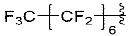 | Hydro carbon group having 1 to 200 carbon atom which may contain a hetero atom |
| 27 | 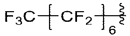 | Hydro carbon group having 1 to 100 carbon atom which may contain a hetero atom |
| 28 | 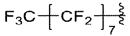 | Hydro carbon group having 1 to 1000 carbon atom which may contain a hetero atom |
| 29 | 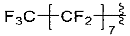 | Hydro carbon group having 1 to 200 carbon atom which may contain a hetero atom |
| 30 | 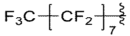 | Hydro carbon group having 1 to 100 carbon atom which may contain a hetero atom |
| 31 | 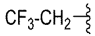 | Hydro carbon group having 1 to 1000 carbon atom which may contain a hetero atom |
| 32 | 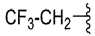 | Hydro carbon group having 1 to 200 carbon atom which may contain a hetero atom |
| 33 | 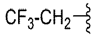 | Hydro carbon group having 1 to 100 carbon atom which may contain a hetero atom |
| 34 | 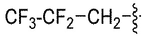 | Hydro carbon group having 1 to 1000 carbon atom which may contain a hetero atom |
| 35 | 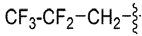 | Hydro carbon group having 1 to 200 carbon atom which may contain a hetero atom |
| 36 | 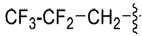 | Hydro carbon group having 1 to 100 carbon atom which may contain a hetero atom |
| 37 | 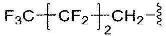 | Hydro carbon group having 1 to 1000 carbon atom which may contain a hetero atom |
| 38 | 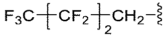 | Hydro carbon group having 1 to 200 carbon atom which may contain a hetero atom |
| 39 | 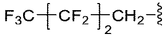 | Hydro carbon group having 1 to 100 carbon atom which may contain a hetero atom |
| 40 | 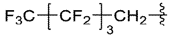 | Hydro carbon group having 1 to 1000 carbon atom which may contain a hetero atom |
| 41 | 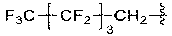 | Hydro carbon group having 1 to 200 carbon atom which may contain a hetero atom |
| 42 | 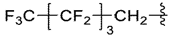 | Hydro carbon group having 1 to 100 carbon atom which may contain a hetero atom |
| 43 | 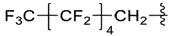 | Hydro carbon group having 1 to 1000 carbon atom which may contain a hetero atom |
| 44 | 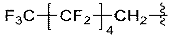 | Hydro carbon group having 1 to 200 carbon atom which may contain a hetero atom |
| 45 | 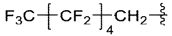 | Hydro carbon group having 1 to 100 carbon atom which may contain a hetero atom |
| 46 | 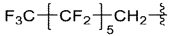 | Hydro carbon group having 1 to 1000 carbon atom which may contain a hetero atom |

**[Table 1-3]**

| Compound No. | R¹ | R² |
|---|---|---|
| 47 | 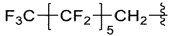 | Hydro carbon group having 1 to 200 carbon atom which may contain a hetero atom |
| 48 | 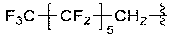 | Hydro carbon group having 1 to 100 carbon atom which may contain a hetero atom |
| 49 | 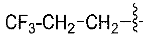 | Hydro carbon group having 1 to 1000 carbon atom which may contain a hetero atom |
| 50 | 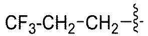 | Hydro carbon group having 1 to 200 carbon atom which may contain a hetero atom |
| 51 | 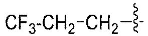 | Hydro carbon group having 1 to 100 carbon atom which may contain a hetero atom |
| 52 | 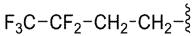 | Hydro carbon group having 1 to 1000 carbon atom which may contain a hetero atom |
| 53 | 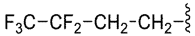 | Hydro carbon group having 1 to 200 carbon atom which may contain a hetero atom |
| 54 | 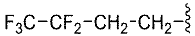 | Hydro carbon group having 1 to 100 carbon atom which may contain a hetero atom |
| 55 | 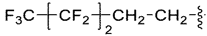 | Hydro carbon group having 1 to 1000 carbon atom which may contain a hetero atom |
| 56 | 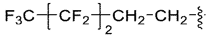 | Hydro carbon group having 1 to 200 carbon atom which may contain a hetero atom |
| 57 | 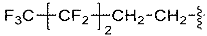 | Hydro carbon group having 1 to 100 carbon atom which may contain a hetero atom |
| 58 | 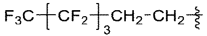 | Hydro carbon group having 1 to 1000 carbon atom which may contain a hetero atom |
| 59 | 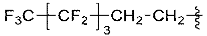 | Hydro carbon group having 1 to 200 carbon atom which may contain a hetero atom |
| 60 | 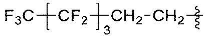 | Hydro carbon group having 1 to 100 carbon atom which may contain a hetero atom |
| 61 | 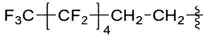 | Hydro carbon group having 1 to 1000 carbon atom which may contain a hetero atom |
| 62 | 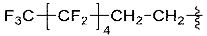 | Hydro carbon group having 1 to 200 carbon atom which may contain a hetero atom |
| 63 | 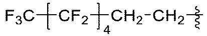 | Hydro carbon group having 1 to 100 carbon atom which may contain a hetero atom |
| 64 | 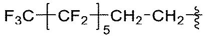 | Hydro carbon group having 1 to 1000 carbon atom which may contain a hetero atom |
| 65 | 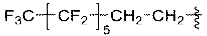 | Hydro carbon group having 1 to 200 carbon atom which may contain a hetero atom |
| 66 | 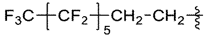 | Hydro carbon group having 1 to 100 carbon atom which may contain a hetero atom |
| 67 | 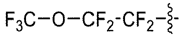 | Hydro carbon group having 1 to 1000 carbon atom which may contain a hetero atom |
| 68 | 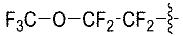 | Hydro carbon group having 1 to 200 carbon atom which may contain a hetero atom |
| 69 | 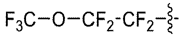 | Hydro carbon group having 1 to 100 carbon atom which may contain a hetero atom |
| 70 | 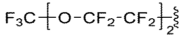 | Hydro carbon group having 1 to 1000 carbon atom which may contain a hetero atom |

**[Table 1-4]**

| Compound No. | R¹ | R² |
|---|---|---|
| 71 | 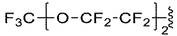 | Hydro carbon group having 1 to 200 carbon atom which may contain a hetero atom |
| 72 | 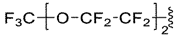 | Hydro carbon group having 1 to 100 carbon atom which may contain a hetero atom |
| 73 | 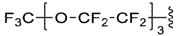 | Hydro carbon group having 1 to 1000 carbon atom which may contain a hetero atom |
| 74 | 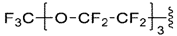 | Hydro carbon group having 1 to 200 carbon atom which may contain a hetero atom |
| 75 | 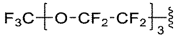 | Hydro carbon group having 1 to 100 carbon atom which may contain a hetero atom |
| 76 | 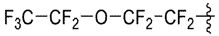 | Hydro carbon group having 1 to 1000 carbon atom which may contain a hetero atom |
| 77 | 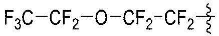 | Hydro carbon group having 1 to 200 carbon atom which may contain a hetero atom |
| 78 | 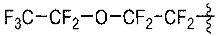 | Hydro carbon group having 1 to 100 carbon atom which may contain a hetero atom |
| 79 | 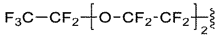 | Hydro carbon group having 1 to 1000 carbon atom which may contain a hetero atom |
| 80 | 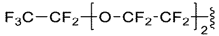 | Hydro carbon group having 1 to 200 carbon atom which may contain a hetero atom |
| 81 | 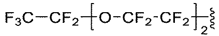 | Hydro carbon group having 1 to 100 carbon atom which may contain a hetero atom |
| 82 | 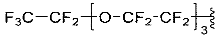 | Hydro carbon group having 1 to 1000 carbon atom which may contain a hetero atom |
| 83 | 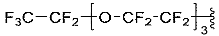 | Hydro carbon group having 1 to 200 carbon atom which may contain a hetero atom |
| 84 | 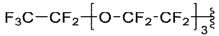 | Hydro carbon group having 1 to 100 carbon atom which may contain a hetero atom |
| 85 | 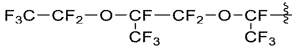 | Hydro carbon group having 1 to 1000 carbon atom which may contain a hetero atom |
| 86 | 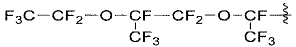 | Hydro carbon group having 1 to 200 carbon atom which may contain a hetero atom |
| 87 | 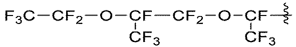 | Hydro carbon group having 1 to 100 carbon atom which may contain a hetero atom |
| 88 | 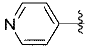 | Hydro carbon group having 1 to 1000 carbon atom which may contain a hetero atom |
| 89 | 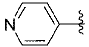 | Hydro carbon group having 1 to 200 carbon atom which may contain a hetero atom |
| 90 | 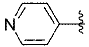 | Hydro carbon group having 1 to 100 carbon atom which may contain a hetero atom |
| 91 | 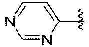 | Hydro carbon group having 1 to 1000 carbon atom which may contain a hetero atom |
| 92 | 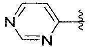 | Hydro carbon group having 1 to 200 carbon atom which may contain a hetero atom |
| 93 | 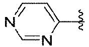 | Hydro carbon group having 1 to 100 carbon atom which may contain a hetero atom |
| 94 | 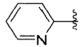 | Hydro carbon group having 1 to 1000 carbon atom which may contain a hetero atom |

**[Table 1-5]**

| Compound No. | R¹ | R² |
|---|---|---|
| 95 | 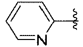 | Hydro carbon group having 1 to 200 carbon atom which may contain a hetero atom |
| 96 | 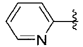 | Hydro carbon group having 1 to 100 carbon atom which may contain a hetero atom |
| 97 | 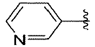 | Hydro carbon group having 1 to 1000 carbon atom which may contain a hetero atom |
| 98 | 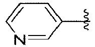 | Hydro carbon group having 1 to 200 carbon atom which may contain a hetero atom |
| 99 | 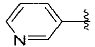 | Hydro carbon group having 1 to 100 carbon atom which may contain a hetero atom |
| 100 | 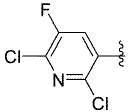 | Hydro carbon group having 1 to 1000 carbon atom which may contain a hetero atom |
| 101 | 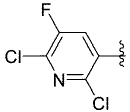 | Hydro carbon group having 1 to 200 carbon atom which may contain a hetero atom |
| 102 | 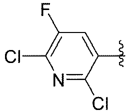 | Hydro carbon group having 1 to 100 carbon atom which may contain a hetero atom |
| 103 | 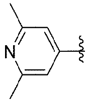 | Hydro carbon group having 1 to 1000 carbon atom which may contain a hetero atom |
| 104 | 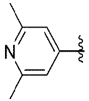 | Hydro carbon group having 1 to 200 carbon atom which may contain a hetero atom |
| 105 | 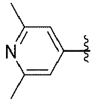 | Hydro carbon group having 1 to 100 carbon atom which may contain a hetero atom |
| 106 | 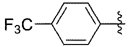 | Hydro carbon group having 1 to 1000 carbon atom which may contain a hetero atom |
| 107 | 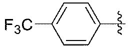 | Hydro carbon group having 1 to 200 carbon atom which may contain a hetero atom |
| 108 | 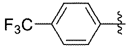 | Hydro carbon group having 1 to 100 carbon atom which may contain a hetero atom |
| 109 | 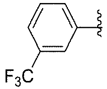 | Hydro carbon group having 1 to 1000 carbon atom which may contain a hetero atom |
| 110 | 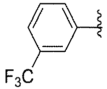 | Hydro carbon group having 1 to 200 carbon atom which may contain a hetero atom |

**[Table 1-6]**

| Compound No. | R¹ | R² |
|---|---|---|
| 111 | 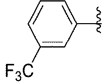 | Hydro carbon group having 1 to 100 carbon atom which may contain a hetero atom |
| 112 | 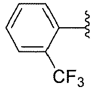 | Hydro carbon group having 1 to 1000 carbon atom which may contain a hetero atom |
| 113 | 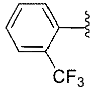 | Hydro carbon group having 1 to 200 carbon atom which may contain a hetero atom |
| 114 | 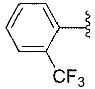 | Hydro carbon group having 1 to 100 carbon atom which may contain a hetero atom |
| 115 | 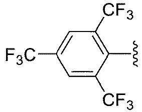 | Hydro carbon group having 1 to 1000 carbon atom which may contain a hetero atom |
| 116 | 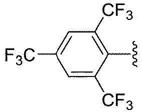 | Hydro carbon group having 1 to 200 carbon atom which may contain a hetero atom |
| 117 | 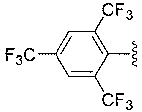 | Hydro carbon group having 1 to 100 carbon atom which may contain a hetero atom |
| 118 | 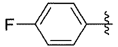 | Hydro carbon group having 1 to 1000 carbon atom which may contain a hetero atom |
| 119 | 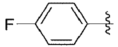 | Hydro carbon group having 1 to 200 carbon atom which may contain a hetero atom |
| 120 | 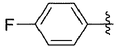 | Hydro carbon group having 1 to 100 carbon atom which may contain a hetero atom |
| 121 | 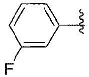 | Hydro carbon group having 1 to 1000 carbon atom which may contain a hetero atom |
| 122 | 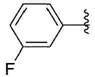 | Hydro carbon group having 1 to 200 carbon atom which may contain a hetero atom |
| 123 | 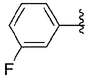 | Hydro carbon group having 1 to 100 carbon atom which may contain a hetero atom |
| 124 | 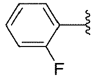 | Hydro carbon group having 1 to 1000 carbon atom which may contain a hetero atom |
| 125 | 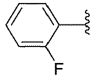 | Hydro carbon group having 1 to 200 carbon atom which may contain a hetero atom |

**[Table 1-7]**

| Compound No. | R¹ | R² |
|---|---|---|
| 126 | 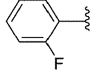 | Hydro carbon group having 1 to 100 carbon atom which may contain a hetero atom |
| 127 | 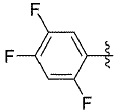 | Hydro carbon group having 1 to 1000 carbon atom which may contain a hetero atom |
| 128 | 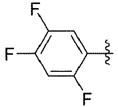 | Hydro carbon group having 1 to 200 carbon atom which may contain a hetero atom |
| 129 | 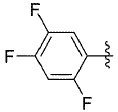 | Hydro carbon group having 1 to 100 carbon atom which may contain a hetero atom |
| 130 | 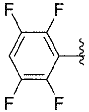 | Hydro carbon group having 1 to 1000 carbon atom which may contain a hetero atom |
| 131 | 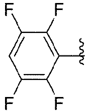 | Hydro carbon group having 1 to 200 carbon atom which may contain a hetero atom |
| 132 | 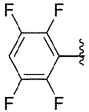 | Hydro carbon group having 1 to 100 carbon atom which may contain a hetero atom |
| 133 | 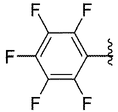 | Hydro carbon group having 1 to 1000 carbon atom which may contain a hetero atom |
| 134 | 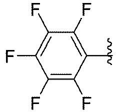 | Hydro carbon group having 1 to 200 carbon atom which may contain a hetero atom |
| 135 | 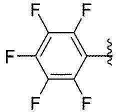 | Hydro carbon group having 1 to 100 carbon atom which may contain a hetero atom |
| 136 | 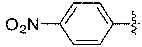 | Hydro carbon group having 1 to 1000 carbon atom which may contain a hetero atom |
| 137 | 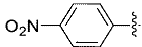 | Hydro carbon group having 1 to 200 carbon atom which may contain a hetero atom |

**[Table 1-8]**

| Compound No. | R¹ | R² |
|---|---|---|
| 138 | 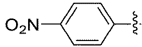 | Hydro carbon group having 1 to 100 carbon atom which may contain a hetero atom |
| 139 | 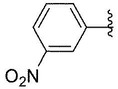 | Hydro carbon group having 1 to 1000 carbon atom which may contain a hetero atom |
| 140 | 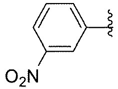 | Hydro carbon group having 1 to 200 carbon atom which may contain a hetero atom |
| 141 | 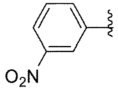 | Hydro carbon group having 1 to 100 carbon atom which may contain a hetero atom |
| 142 | 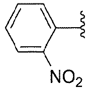 | Hydro carbon group having 1 to 1000 carbon atom which may contain a hetero atom |
| 143 | 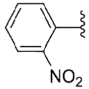 | Hydro carbon group having 1 to 200 carbon atom which may contain a hetero atom |
| 144 | 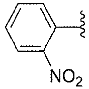 | Hydro carbon group having 1 to 100 carbon atom which may contain a hetero atom |
| 145 | 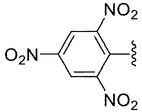 | Hydro carbon group having 1 to 1000 carbon atom which may contain a hetero atom |
| 146 | 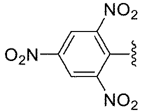 | Hydro carbon group having 1 to 200 carbon atom which may contain a hetero atom |
| 147 | 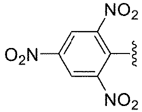 | Hydro carbon group having 1 to 100 carbon atom which may contain a hetero atom |
| 148 | 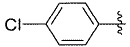 | Hydro carbon group having 1 to 1000 carbon atom which may contain a hetero atom |
| 149 | 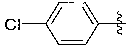 | Hydro carbon group having 1 to 200 carbon atom which may contain a hetero atom |
| 150 | 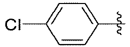 | Hydro carbon group having 1 to 100 carbon atom which may contain a hetero atom |
| 151 | 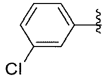 | Hydro carbon group having 1 to 1000 carbon atom which may contain a hetero atom |
| 152 | 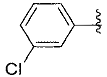 | Hydro carbon group having 1 to 200 carbon atom which may contain a hetero atom |

**[Table 1-9]**

| Compound No. | R¹ | R² |
|---|---|---|
| 153 | 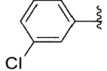 | Hydro carbon group having 1 to 100 carbon atom which may contain a hetero atom |
| 154 | 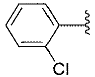 | Hydro carbon group having 1 to 1000 carbon atom which may contain a hetero atom |
| 155 | 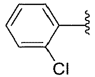 | Hydro carbon group having 1 to 200 carbon atom which may contain a hetero atom |
| 156 | 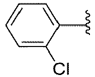 | Hydro carbon group having 1 to 100 carbon atom which may contain a hetero atom |
| 157 | 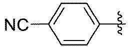 | Hydro carbon group having 1 to 1000 carbon atom which may contain a hetero atom |
| 158 | 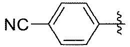 | Hydro carbon group having 1 to 200 carbon atom which may contain a hetero atom |
| 159 | 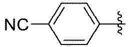 | Hydro carbon group having 1 to 100 carbon atom which may contain a hetero atom |
| 160 | 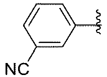 | Hydro carbon group having 1 to 1000 carbon atom which may contain a hetero atom |
| 161 | 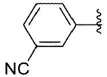 | Hydro carbon group having 1 to 200 carbon atom which may contain a hetero atom |
| 162 | 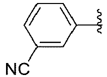 | Hydro carbon group having 1 to 100 carbon atom which may contain a hetero atom |
| 163 | 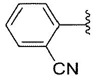 | Hydro carbon group having 1 to 1000 carbon atom which may contain a hetero atom |
| 164 | 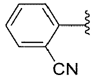 | Hydro carbon group having 1 to 200 carbon atom which may contain a hetero atom |
| 165 | 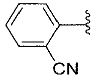 | Hydro carbon group having 1 to 100 carbon atom which may contain a hetero atom |
| 166 | 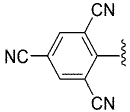 | Hydro carbon group having 1 to 1000 carbon atom which may contain a hetero atom |
| 167 | 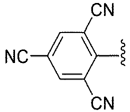 | Hydro carbon group having 1 to 200 carbon atom which may contain a hetero atom |

**[Table 1-10]**

| Compound No. | R¹ | R² |
|---|---|---|
| 168 | 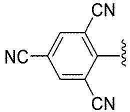 | Hydro carbon group having 1 to 100 carbon atom which may contain a hetero atom |
| 169 | 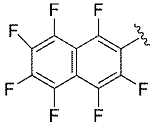 | Hydro carbon group having 1 to 1000 carbon atom which may contain a hetero atom |
| 170 | 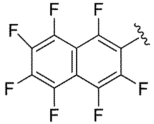 | Hydro carbon group having 1 to 200 carbon atom which may contain a hetero atom |
| 171 | 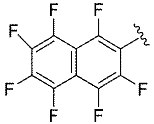 | Hydro carbon group having 1 to 100 carbon atom which may contain a hetero atom |
| 172 | 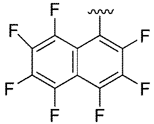 | Hydro carbon group having 1 to 1000 carbon atom which may contain a hetero atom |
| 173 | 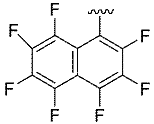 | Hydro carbon group having 1 to 200 carbon atom which may contain a hetero atom |
| 174 | 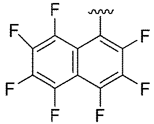 | Hydro carbon group having 1 to 100 carbon atom which may contain a hetero atom |
| 175 | 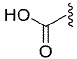 | Hydro carbon group having 1 to 1000 carbon atom which may contain a hetero atom |
| 176 | 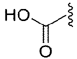 | Hydro carbon group having 1 to 200 carbon atom which may contain a hetero atom |
| 177 | 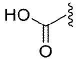 | Hydro carbon group having 1 to 100 carbon atom which may contain a hetero atom |
| 178 | 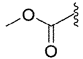 | Hydro carbon group having 1 to 1000 carbon atom which may contain a hetero atom |
| 179 | 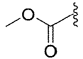 | Hydro carbon group having 1 to 200 carbon atom which may contain a hetero atom |

**[Table 1-11]**

| Compound No. | R¹ | R² |
|---|---|---|
| 180 | 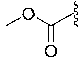 | Hydro carbon group having 1 to 100 carbon atom which may contain a hetero atom |
| 181 | 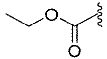 | Hydro carbon group having 1 to 1000 carbon atom which may contain a hetero atom |
| 182 | 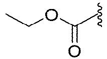 | Hydro carbon group having 1 to 200 carbon atom which may contain a hetero atom |
| 183 | 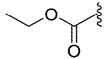 | Hydro carbon group having 1 to 100 carbon atom which may contain a hetero atom |
| 184 | 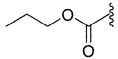 | Hydro carbon group having 1 to 1000 carbon atom which may contain a hetero atom |
| 185 | 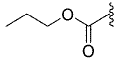 | Hydro carbon group having 1 to 200 carbon atom which may contain a hetero atom |
| 186 | 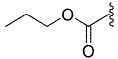 | Hydro carbon group having 1 to 100 carbon atom which may contain a hetero atom |
| 187 | 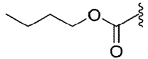 | Hydro carbon group having 1 to 1000 carbon atom which may contain a hetero atom |
| 188 | 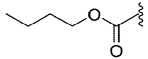 | Hydro carbon group having 1 to 200 carbon atom which may contain a hetero atom |
| 189 | 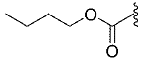 | Hydro carbon group having 1 to 100 carbon atom which may contain a hetero atom |
| 190 | 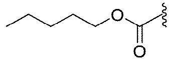 | Hydro carbon group having 1 to 1000 carbon atom which may contain a hetero atom |
| 191 | 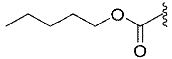 | Hydro carbon group having 1 to 200 carbon atom which may contain a hetero atom |
| 192 | 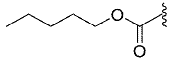 | Hydro carbon group having 1 to 100 carbon atom which may contain a hetero atom |
| 193 | 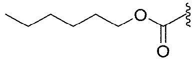 | Hydro carbon group having 1 to 1000 carbon atom which may contain a hetero atom |
| 194 | 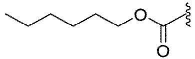 | Hydro carbon group having 1 to 200 carbon atom which may contain a hetero atom |
| 195 | 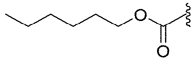 | Hydro carbon group having 1 to 100 carbon atom which may contain a hetero atom |
| 196 | 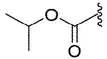 | Hydro carbon group having 1 to 1000 carbon atom which may contain a hetero atom |
| 197 | 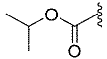 | Hydro carbon group having 1 to 200 carbon atom which may contain a hetero atom |
| 198 | 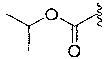 | Hydro carbon group having 1 to 100 carbon atom which may contain a hetero atom |

**[Table 1-12]**

| Compound No. | R¹ | R² |
|---|---|---|
| 199 | 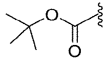 | Hydro carbon group having 1 to 1000 carbon atom which may contain a hetero atom |
| 200 | 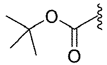 | Hydro carbon group having 1 to 200 carbon atom which may contain a hetero atom |
| 201 | 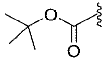 | Hydro carbon group having 1 to 100 carbon atom which may contain a hetero atom |
| 202 | 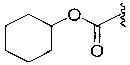 | Hydro carbon group having 1 to 1000 carbon atom which may contain a hetero atom |
| 203 | 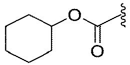 | Hydro carbon group having 1 to 200 carbon atom which may contain a hetero atom |
| 204 | 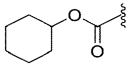 | Hydro carbon group having 1 to 100 carbon atom which may contain a hetero atom |
| 205 | 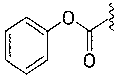 | Hydro carbon group having 1 to 1000 carbon atom which may contain a hetero atom |
| 206 | 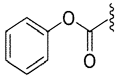 | Hydro carbon group having 1 to 200 carbon atom which may contain a hetero atom |
| 207 | 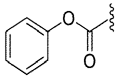 | Hydro carbon group having 1 to 100 carbon atom which may contain a hetero atom |
| 208 | 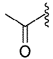 | Hydro carbon group having 1 to 1000 carbon atom which may contain a hetero atom |
| 209 | 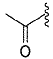 | Hydro carbon group having 1 to 200 carbon atom which may contain a hetero atom |
| 210 | 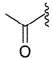 | Hydro carbon group having 1 to 100 carbon atom which may contain a hetero atom |
| 211 | 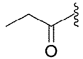 | Hydro carbon group having 1 to 1000 carbon atom which may contain a hetero atom |
| 212 | 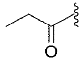 | Hydro carbon group having 1 to 200 carbon atom which may contain a hetero atom |
| 213 | 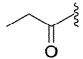 | Hydro carbon group having 1 to 100 carbon atom which may contain a hetero atom |
| 214 | 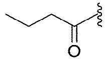 | Hydro carbon group having 1 to 1000 carbon atom which may contain a hetero atom |
| 2 1 5 | 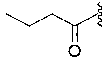 | Hydro carbon group having 1 to 200 carbon atom which may contain a hetero atom |
| 2 1 6 | 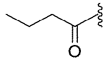 | Hydro carbon group having 1 to 100 carbon atom which may contain a hetero atom |
| 217 | 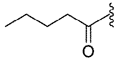 | Hydro carbon group having 1 to 1000 carbon atom which may contain a hetero atom |

**[Table 1-13]**

| Compound No. | R¹ | R² |
|---|---|---|
| 218 | 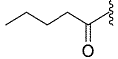 | Hydro carbon group having 1 to 200 carbon atom which may contain a hetero atom |
| 219 | 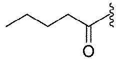 | Hydro carbon group having 1 to 100 carbon atom which may contain a hetero atom |
| 2 2 0 | 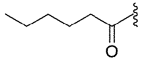 | Hydro carbon group having 1 to 1000 carbon atom which may contain a hetero atom |
| 2 2 1 | 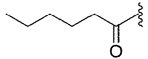 | Hydro carbon group having 1 to 200 carbon atom which may contain a hetero atom |
| 222 | 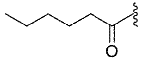 | Hydro carbon group having 1 to 100 carbon atom which may contain a hetero atom |
| 223 | 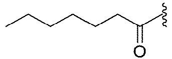 | Hydro carbon group having 1 to 1000 carbon atom which may contain a hetero atom |
| 224 | 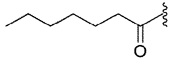 | Hydro carbon group having 1 to 200 carbon atom which may contain a hetero atom |
| 225 | 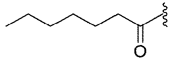 | Hydro carbon group having 1 to 100 carbon atom which may contain a hetero atom |
| 226 | 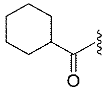 | Hydro carbon group having 1 to 1000 carbon atom which may contain a hetero atom |
| 227 | 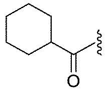 | Hydro carbon group having 1 to 200 carbon atom which may contain a hetero atom |
| 228 | 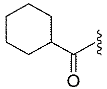 | Hydro carbon group having 1 to 100 carbon atom which may contain a hetero atom |
| 2 2 9 | 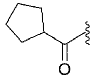 | Hydro carbon group having 1 to 1000 carbon atom which may contain a hetero atom |
| 230 | 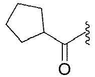 | Hydro carbon group having 1 to 200 carbon atom which may contain a hetero atom |
| 231 | 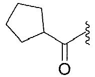 | Hydro carbon group having 1 to 100 carbon atom which may contain a hetero atom |
| 232 | 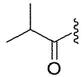 | Hydro carbon group having 1 to 1000 carbon atom which may contain a hetero atom |
| 2 3 3 | 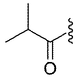 | Hydro carbon group having 1 to 200 carbon atom which may contain a hetero atom |
| 234 | 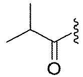 | Hydro carbon group having 1 to 100 carbon atom which may contain a hetero atom |

**[Table 1-14]**

| Compound No. | R¹ | R² |
|---|---|---|
| 235 | 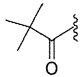 | Hydro carbon group having 1 to 1000 carbon atom which may contain a hetero atom |
| 236 | 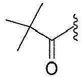 | Hydro carbon group having 1 to 200 carbon atom which may contain a hetero atom |
| 237 | 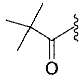 | Hydro carbon group having 1 to 100 carbon atom which may contain a hetero atom |
| 238 | 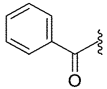 | Hydro carbon group having 1 to 1000 carbon atom which may contain a hetero atom |
| 239 | 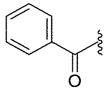 | Hydro carbon group having 1 to 200 carbon atom which may contain a hetero atom |
| 240 | 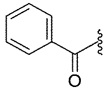 | Hydro carbon group having 1 to 100 carbon atom which may contain a hetero atom |
| 241 | 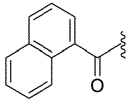 | Hydro carbon group having 1 to 1000 carbon atom which may contain a hetero atom |
| 242 | 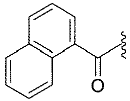 | Hydro carbon group having 1 to 200 carbon atom which may contain a hetero atom |
| 243 | 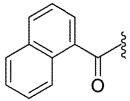 | Hydro carbon group having 1 to 100 carbon atom which may contain a hetero atom |
| 244 | 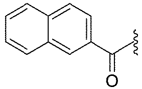 | Hydro carbon group having 1 to 1000 carbon atom which may contain a hetero atom |
| 245 | 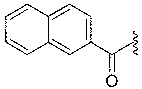 | Hydro carbon group having 1 to 200 carbon atom which may contain a hetero atom |
| 246 | 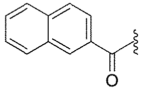 | Hydro carbon group having 1 to 100 carbon atom which may contain a hetero atom |
| 247 | 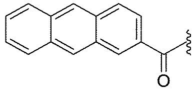 | Hydro carbon group having 1 to 1000 carbon atom which may contain a hetero atom |
| 248 | 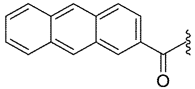 | Hydro carbon group having 1 to 200 carbon atom which may contain a hetero atom |

**[Table 1-15]**

| Compound No. | R¹ | R² |
|---|---|---|
| 249 | 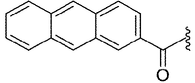 | Hydro carbon group having 1 to 100 carbon atom which may contain a hetero atom |
| 250 | 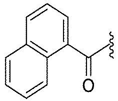 | Hydro carbon group having 1 to 1000 carbon atom which may contain a hetero atom |
| 251 | 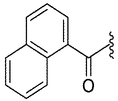 | Hydro carbon group having 1 to 200 carbon atom which may contain a hetero atom |
| 252 | 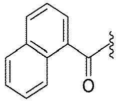 | Hydro carbon group having 1 to 100 carbon atom which may contain a hetero atom |
| 253 | 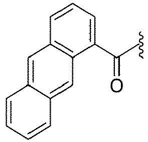 | Hydro carbon group having 1 to 1000 carbon atom which may contain a hetero atom |
| 254 | 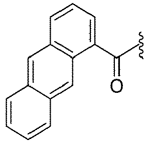 | Hydro carbon group having 1 to 200 carbon atom which may contain a hetero atom |
| 255 | 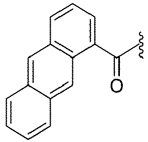 | Hydro carbon group having 1 to 100 carbon atom which may contain a hetero atom |
| 256 | 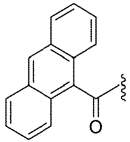 | Hydro carbon group having 1 to 1000 carbon atom which may contain a hetero atom |
| 257 | 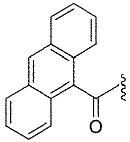 | Hydro carbon group having 1 to 200 carbon atom which may contain a hetero atom |
| 258 | 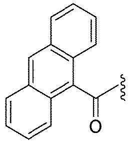 | Hydro carbon group having 1 to 100 carbon atom which may contain a hetero atom |

**[Table 1-16]**

| Compound No. | R¹ | R² |
|---|---|---|
| 259 | 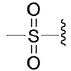 | Hydro carbon group having 1 to 1000 carbon atom which may contain a hetero atom |
| 260 | 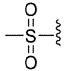 | Hydro carbon group having 1 to 200 carbon atom which may contain a hetero atom |
| 261 | 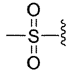 | Hydro carbon group having 1 to 100 carbon atom which may contain a hetero atom |
| 262 | 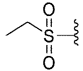 | Hydro carbon group having 1 to 1000 carbon atom which may contain a hetero atom |
| 263 | 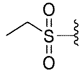 | Hydro carbon group having 1 to 200 carbon atom which may contain a hetero atom |
| 264 | 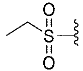 | Hydro carbon group having 1 to 100 carbon atom which may contain a hetero atom |
| 265 | 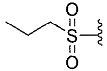 | Hydro carbon group having 1 to 1000 carbon atom which may contain a hetero atom |
| 266 | 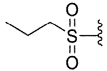 | Hydro carbon group having 1 to 200 carbon atom which may contain a hetero atom |
| 267 | 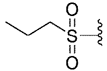 | Hydro carbon group having 1 to 100 carbon atom which may contain a hetero atom |
| 268 | 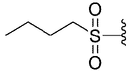 | Hydro carbon group having 1 to 1000 carbon atom which may contain a hetero atom |
| 269 | 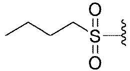 | Hydro carbon group having 1 to 200 carbon atom which may contain a hetero atom |
| 270 | 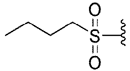 | Hydro carbon group having 1 to 100 carbon atom which may contain a hetero atom |
| 271 | 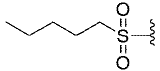 | Hydro carbon group having 1 to 1000 carbon atom which may contain a hetero atom |
| 272 | 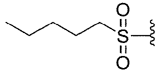 | Hydro carbon group having 1 to 200 carbon atom which may contain a hetero atom |
| 273 | 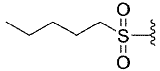 | Hydro carbon group having 1 to 100 carbon atom which may contain a hetero atom |
| 274 | 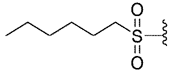 | Hydro carbon group having 1 to 1000 carbon atom which may contain a hetero atom |

**[Table 1-17]**

| Compound No. | R¹ | R² |
|---|---|---|
| 275 | 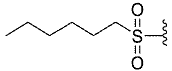 | Hydro carbon group having 1 to 200 carbon atom which may contain a hetero atom |
| 276 | 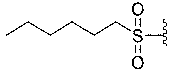 | Hydro carbon group having 1 to 100 carbon atom which may contain a hetero atom |
| 277 | 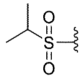 | Hydro carbon group having 1 to 1000 carbon atom which may contain a hetero atom |
| 278 | 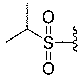 | Hydro carbon group having 1 to 200 carbon atom which may contain a hetero atom |
| 279 | 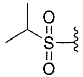 | Hydro carbon group having 1 to 100 carbon atom which may contain a hetero atom |
| 280 | 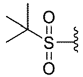 | Hydro carbon group having 1 to 1000 carbon atom which may contain a hetero atom |
| 281 | 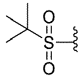 | Hydro carbon group having 1 to 200 carbon atom which may contain a hetero atom |
| 282 | 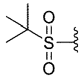 | Hydro carbon group having 1 to 100 carbon atom which may contain a hetero atom |
| 283 | 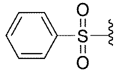 | Hydro carbon group having 1 to 1000 carbon atom which may contain a hetero atom |
| 284 | 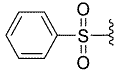 | Hydro carbon group having 1 to 200 carbon atom which may contain a hetero atom |
| 285 | 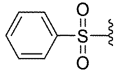 | Hydro carbon group having 1 to 100 carbon atom which may contain a hetero atom |
| 286 | 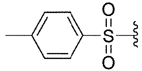 | Hydro carbon group having 1 to 1000 carbon atom which may contain a hetero atom |
| 287 | 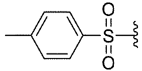 | Hydro carbon group having 1 to 200 carbon atom which may contain a hetero atom |
| 288 | 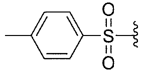 | Hydro carbon group having 1 to 100 carbon atom which may contain a hetero atom |
| 289 | 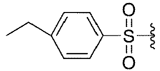 | Hydro carbon group having 1 to 1000 carbon atom which may contain a hetero atom |
| 290 | 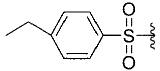 | Hydro carbon group having 1 to 200 carbon atom which may contain a hetero atom |

**[Table 1-18]**

| Compound No. | R¹ | R² |
|---|---|---|
| 291 | 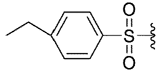 | Hydro carbon group having 1 to 100 carbon atom which may contain a hetero atom |
| 292 | 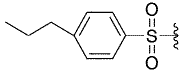 | Hydro carbon group having 1 to 1000 carbon atom which may contain a hetero atom |
| 293 | 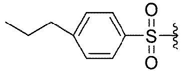 | Hydro carbon group having 1 to 200 carbon atom which may contain a hetero atom |
| 294 | 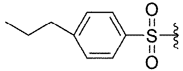 | Hydro carbon group having 1 to 100 carbon atom which may contain a hetero atom |
| 295 | 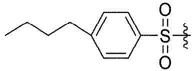 | Hydro carbon group having 1 to 1000 carbon atom which may contain a hetero atom |
| 296 | 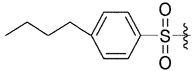 | Hydro carbon group having 1 to 200 carbon atom which may contain a hetero atom |
| 297 | 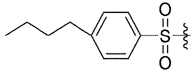 | Hydro carbon group having 1 to 100 carbon atom which may contain a hetero atom |
| 298 | 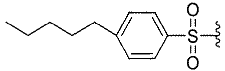 | Hydro carbon group having 1 to 1000 carbon atom which may contain a hetero atom |
| 299 | 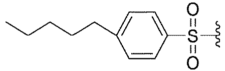 | Hydro carbon group having 1 to 200 carbon atom which may contain a hetero atom |
| 300 | 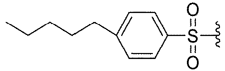 | Hydro carbon group having 1 to 100 carbon atom which may contain a hetero atom |
| 301 | 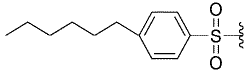 | Hydro carbon group having 1 to 1000 carbon atom which may contain a hetero atom |
| 302 | 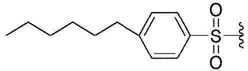 | Hydro carbon group having 1 to 200 carbon atom which may contain a hetero atom |
| 303 | 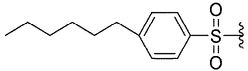 | Hydro carbon group having 1 to 100 carbon atom which may contain a hetero atom |
| 304 | 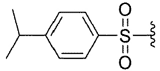 | Hydro carbon group having 1 to 1000 carbon atom which may contain a hetero atom |
| 305 | 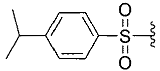 | Hydro carbon group having 1 to 200 carbon atom which may contain a hetero atom |
| 306 | 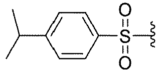 | Hydro carbon group having 1 to 100 carbon atom which may contain a hetero atom |

**[Table 1-19]**

| Compound No. | R¹ | R² |
|---|---|---|
| 307 | 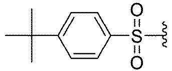 | Hydro carbon group having 1 to 1000 carbon atom which may contain a hetero atom |
| 308 | 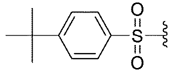 | Hydro carbon group having 1 to 200 carbon atom which may contain a hetero atom |
| 309 | 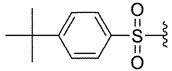 | Hydro carbon group having 1 to 100 carbon atom which may contain a hetero atom |
| 310 | 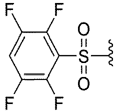 | Hydro carbon group having 1 to 1000 carbon atom which may contain a hetero atom |
| 311 | 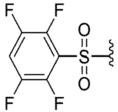 | Hydro carbon group having 1 to 200 carbon atom which may contain a hetero atom |
| 312 | 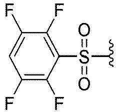 | Hydro carbon group having 1 to 100 carbon atom which may contain a hetero atom |
| 313 | 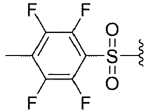 | Hydro carbon group having 1 to 1000 carbon atom which may contain a hetero atom |
| 314 | 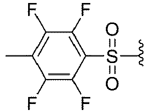 | Hydro carbon group having 1 to 200 carbon atom which may contain a hetero atom |
| 315 | 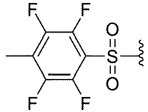 | Hydro carbon group having 1 to 100 carbon atom which may contain a hetero atom |
| 316 | 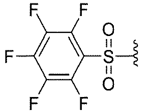 | Hydro carbon group having 1 to 1000 carbon atom which may contain a hetero atom |
| 317 | 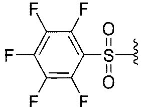 | Hydro carbon group having 1 to 200 carbon atom which may contain a hetero atom |

**[Table 1-20]**

| Compound No. | R¹ | R² |
|---|---|---|
| 318 | 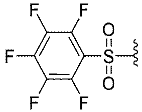 | Hydro carbon group having 1 to 100 carbon atom which may contain a hetero atom |
| 319 | 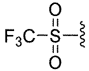 | Hydro carbon group having 1 to 1000 carbon atom which may contain a hetero atom |
| 320 | 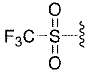 | Hydro carbon group having 1 to 200 carbon atom which may contain a hetero atom |
| 321 | 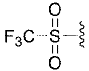 | Hydro carbon group having 1 to 100 carbon atom which may contain a hetero atom |
| 322 | 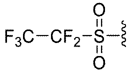 | Hydro carbon group having 1 to 1000 carbon atom which may contain a hetero atom |
| 323 | 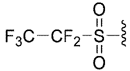 | Hydro carbon group having 1 to 200 carbon atom which may contain a hetero atom |
| 324 | 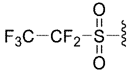 | Hydro carbon group having 1 to 100 carbon atom which may contain a hetero atom |
| 325 | 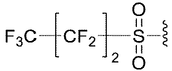 | Hydro carbon group having 1 to 1000 carbon atom which may contain a hetero atom |
| 326 | 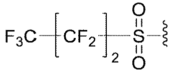 | Hydro carbon group having 1 to 200 carbon atom which may contain a hetero atom |
| 327 | 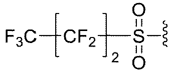 | Hydro carbon group having 1 to 100 carbon atom which may contain a hetero atom |
| 328 | 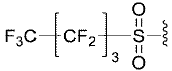 | Hydro carbon group having 1 to 1000 carbon atom which may contain a hetero atom |
| 329 | 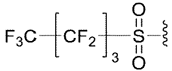 | Hydro carbon group having 1 to 200 carbon atom which may contain a hetero atom |
| 330 | 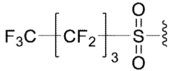 | Hydro carbon group having 1 to 100 carbon atom which may contain a hetero atom |

**[Table 1-21]**

| Compound No. | R¹ | R² |
|---|---|---|
| 3 3 1 | 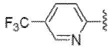 | Hydro carbon group having 1 to 1000 carbon atom which may contain a hetero atom |
| 3 3 2 | 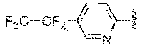 | Hydro carbon group having 1 to 1000 carbon atom which may contain a hetero atom |
| 3 3 3 | 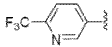 | Hydro carbon group having 1 to 1000 carbon atom which may contain a hetero atom |
| 3 3 4 | 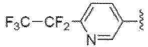 | Hydro carbon group having 1 to 1000 carbon atom which may contain a hetero atom |

### INDUSTRIAL APPLICABILITY

By using the pyrazolone compound of the present invention, it is possible to introduce drugs to formyl groups on antibodies under mild conditions, and prepare chemically and biochemically stable antibody-drug complexes, and thus the present invention is expected to contribute to drug development. In addition, it is possible to form stable modifications with formyl groups at the reducing ends of glycans by the pyrazolone compounds of the present invention. Therefore, it is possible to quantitatively and qualitatively analyze sugar chain. Furthermore, it is possible to analyze the structure in detail by enzymatic reactions. Therefore, the present invention is expected to contribute to biology and medicine, especially to diagnosis and pathological analysis.

## Claims

1. A pyrazolone compound represented by the following formula (1): 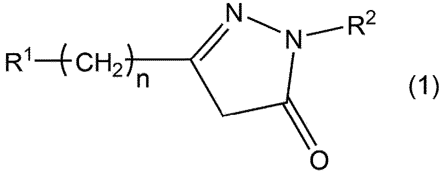 wherein R¹ is an electron withdrawing group selected from the group consisting of one or more selected from the group consisting of a halogen atom, a nitro group, a cyano group, a haloalkyl group having 1 to 10 carbon atoms (one or more halogen atoms attached to the carbon atom at position 1), a perfluoropolyether group having 2 to 10 carbon atoms, a carboxy group, a substituted aryl group having 6-60 carbon atoms (wherein a benzene ring attached to the side of pyrazolone skeleton has at least one substituent, and said substituent is one or more group selected from a halogen atom, a nitro group, a cyano group, a haloalkyl group having 1 to 10 carbon atoms (one or more halogen atoms are attached to the carbon atom at position 1), perfluoropolyether group having 2 to 10 carbon atoms, a carboxy group, an acyl group having 2 to 61 carbon atoms, an alkoxycarbonyl group having 1 to 60 carbon atoms, an alkoxy group having 1 to 60 carbon atoms, and alkyl group having 1 to 60 carbon atoms; and when the substituent is the alkoxy group having 1 to 60 carbon atoms or the alkyl group having 1 to 60 carbon atoms, the position of the substituent is the meta-position of the benzene ring attached to the side of the pyrazolone skeleton), and the following formulas (2) to (7): 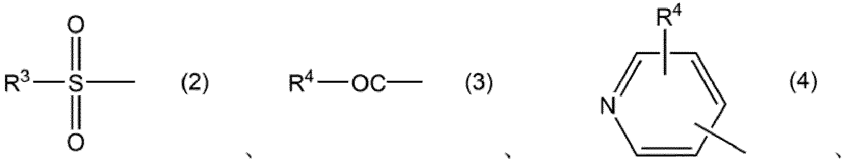 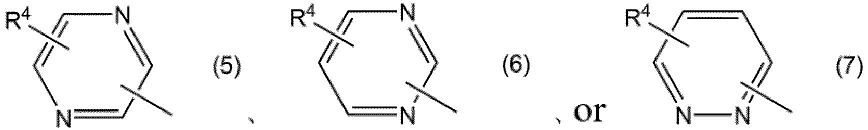 wherein
R³ is a halogen atom, a nitro group, a cyano group, a haloalkyl group having 1 to 10 carbon atoms, a perfluoropolyether group having 2 to 10 carbon atoms, a carboxy group, an optionally substituted alkyl group having 1 to 60 carbon atoms, an optionally substituted alkenyl group having 1 to 60 carbon atoms, an optionally substituted alkynyl group having 1 to 60 carbon atoms, an optionally substituted aralkyl group having 1 to 60 carbon atoms, an optionally substituted aryl group having 6 to 60 carbon atoms, an optionally substituted alkoxy group having 1 to 60 carbon atoms, an optionally substituted alkenyloxy group having 1 to 60 carbon atoms, an optionally substituted alkynyloxy group having 1 to 60 carbon atoms, an optionally substituted aralkyloxy group having 1 to 60 carbon atoms, and/or an optionally substituted aryloxy group having 6 to 60 carbon atoms, and
R⁴ is a halogen atom, a nitro group, a cyano group, a haloalkyl group having 1 to 10 carbon atoms, a perfluoropolyether group having 2 to 10 carbon atoms, a carboxy group, an optionally substituted alkyl group having 1 to 60 carbon atoms, an optionally substituted alkenyl group having 1 to 60 carbon atoms, an optionally substituted alkynyl group having 1 to 60 carbon atoms, an optionally substituted aralkyl group having 1 to 60 carbon atoms, an optionally substituted aryl group having 6 to 60 carbon atoms, an optionally substituted alkoxy group having 1 to 60 carbon atoms, an optionally substituted alkenyloxy group having 1 to 60 carbon atoms, an optionally substituted alkynyloxy group having 1 to 60 carbon atoms, an optionally substituted aralkyloxy group having 1 to 60 carbon atoms, an optionally substituted aryloxy group having 6 to 60 carbon atoms, an optionally substituted alkyl carbonyl group having 1 to 60 carbon atoms, an optionally substituted alkenyl carbonyl group having 1 to 60 carbon atoms, an optionally substituted alkynyl carbonyl group having 1 to 60 carbon atoms, an optionally substituted alalkyl carbonyl group having 1 to 60 carbon atoms, an optionally substituted arylcarbonyl group having 6 to 60 carbon atoms, an optionally substituted alkoxycarbonyl group having 1 to 60 carbon atoms, an optionally substituted alkenyloxycarbonyl group having 1 to 60 carbon atoms, an optionally substituted alkynyloxycarbonyl group having 1 to 60 carbon atoms, an optionally substituted aralkyloxycarbonyl group having 1 to 60 carbon atoms, and/or an optionally substituted aryloxycarbonyl group having 6 to 60 carbon atoms, and R⁴ is 0 to 4 in the formula (4), and R⁴ is 0 to 3 in the formulas (5) to (7), and
R² is a hydrocarbon group having 1 to 1000 carbon atoms, which may contain a heteroatom, and n is an integer of 0 to 2.

2. A pyrazolonating agent for formyl group-containing compound, comprising the pyrazolone compound according to claim 1.

3. A pyrazolonation method for formyl group-containing compound, **characterized in that** a formyl group-containing compound is brought into contact with the pyrazolone compound according to claim 1.

4. A method for stabilizing a pyrazolonated compound, **characterized in that** a formyl group-containing compound is brought into contact with the pyrazolone compound according to claim 1.

5. A synthesis method for pyrazolonated compound, **characterized in that** a formyl group-containing compound is brought into contact with the pyrazolone compound according to claim 1.

6. A pyrazolonated compound obtained by the synthesis method according to claim 5.
